(19) Europäisches Patentamt
European Patent Office
Office européen des brevets

(11) Numéro de publication: 0 332 528 A1

(12) **DEMANDE DE BREVET EUROPEEN**

(21) Numéro de dépôt: 89400630.3

(22) Date de dépôt: 07.03.89

(51) Int. Cl.4: C 07 D 401/06, C 07 D 209/46, C 07 D 209/48, C 07 D 209/50, C 07 D 401/14

(30) Priorité: 08.03.88 FR 8802918

(43) Date de publication de la demande: 13.09.89 Bulletin 89/37

(84) Etats contractants désignés: AT BE CH DE ES FR GB GR IT LI LU NL SE

(71) Demandeur: RHONE-POULENC SANTE
20, avenue Raymond Aron
F-92160 Antony (FR)

(72) Inventeur: Comte, Marie-Thérèse
1 Allée Costes et Bellonte
F-94550 Chevilly Larue (FR)

Gueremy, Claude
3 Rue Daumesnil
F-78800 Houilles (FR)

Ponsinet, Gérard
7 Rue de Grand Champ
F-94370 Sucy-en-Brie (FR)

(74) Mandataire: Savina, Jacques et al
RHONE-POULENC SANTE Service Brevets 25, Quai Paul Doumer
F-92408 Courbevoie Cédex (FR)

Claims for the following Contracting States: ES + GR.

(54) **Dérivés d'isoindolinone, leurs procédés de préparation et les médicaments les contenant.**

(57) Composés de formule :

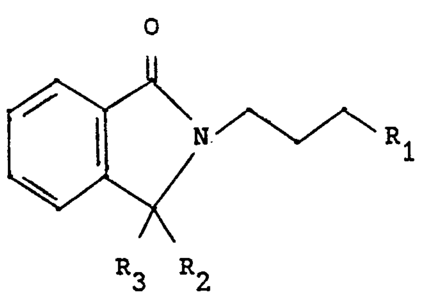

(I)

dans laquelle :
- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un atome d'hydrogène ou un radical alcoxy contenant 1 ou 2 atomes de carbone, hydroxy, alkyle, alkylthio, alkylcarbonyloxy, phénylalkylcarbonyloxy, phénylalkyle ou $-NR_4R_5$ dans lequel $R_4$ représente un atome d'hydrogène ou un radical alkyle et $R_5$ représente un radical alkyle, phényle, phényle substitué par un atome d'halogène ou pyridyle et $R_3$ représente un atome d'hydrogène ou bien $R_2$ représente un radical phényle et $R_3$ représente un atome d'hydrogène ou un radical hydroxy ;
- soit $R_1$ représente un radical phényl-4 pipérazinyl-1 dont le noyau phényle est substitué en position-4 par un atome d'halogène ou un radical hydroxy, $R_2$ représente un radical alcoxy et $R_3$ représente un atome d'hydrogène ;
étant entendu que lorsque $R_1$ représente le radical phényl-4 tétrahydro-1,2,3,6 pyridyl-1, $R_2$ n'est pas le radical hydroxy et que, sauf mention contraire, les radicaux alkyle et alcoxy et les portions alkyle et alcoxy contiennent 1 à 4 atomes de carbone en chaîne droite ou ramifiée,
et leurs sels d'addition avec un acide minéral ou organique, leurs procédés de préparation et les médicaments les contenant.

EP 0 332 528 A1

**Description**

## DERIVES D'ISOINDOLINONE LEURS PROCEDES DE PREPARATION ET LES MEDICAMENTS LES CONTENANT

La présente invention concerne des dérivés d'isoindolinone de formule :

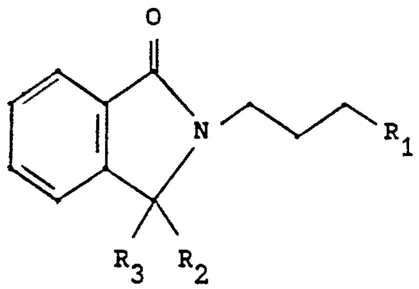

(I)

leurs procédés de préparation et les médicaments les contenant.

Dans la formule (I) :

- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un atome d'hydrogène ou un radical alcoxy contenant 1 ou 2 atomes de carbone, hydroxy, alkyle, alkylthio, alkylcarbonyloxy, phénylalkylcarbonyloxy, phénylalkyle ou $-NR_4R_5$ dans lequel $R_4$ représente un atome d'hydrogène ou un radical alkyle et $R_5$ représente un radical alkyle, phényle, phényle substitué par un atome d'halogène ou pyridyle et $R_3$ représente un atome d'hydrogène ou bien $R_2$ représente un radical phényle et $R_3$ représente un atome d'hydrogène ou un radical hydroxy ;
- soit $R_1$ représente un radical phényl-4 pipérazinyl-1 dont le noyau phényle est substitué en position-4 par un atome d'halogène ou un radical hydroxy, $R_2$ représente un radical alcoxy et $R_3$ représente un atome d'hydrogène ;

étant entendu que lorsque $R_1$ représente le radical phényl-4 tétrahydro-1,2,3,6 pyridyl-1, $R_2$ n'est pas le radical hydroxy, et leurs sels d'addition avec un acide minéral ou organique.

Sauf mention contraire dans les définitions qui précèdent et celles qui seront citées ci-après, les radicaux alkyle et alcoxy et les portions alkyle et alcoxy contiennent 1 à 4 atomes de carbone en chaîne droite ou ramifiée et les atomes d'halogène sont, de préférence, les atomes de chlore et de fluor.

Les composés de formule (I) pour lesquels $R_2$ représente un radical alcoxy et $R_1$, $R_3$ sont définis comme précédemment peuvent être préparés par alkylation d'un dérivé de formule :

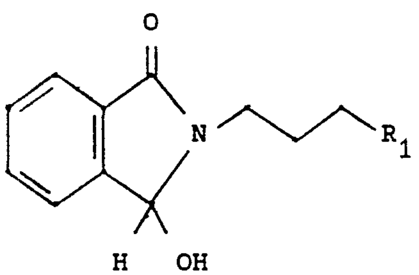

(II)

dans laquelle $R_1$ a les mêmes significations que dans la formule (I).

Cette alkylation s'effectue généralement au moyen d'un alcool aliphatique comportant 1 à 4 atomes de carbone, en présence d'un acide minéral tel que l'acide sulfurique à une température comprise entre 20°C et la température d'ébullition de l'alcool.

Le composé de formule (I) pour lequel $R_2$ représente un radical hydroxy et $R_1$, $R_3$ sont définis comme précédemment et les dérivés de formule (II) non compris dans la formule (I) peuvent être préparés par réduction d'un dérivé de formule :

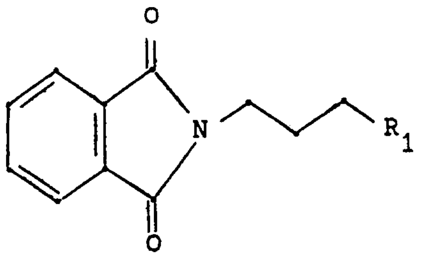

(III)

dans laquelle $R_1$ représente un radical phényl-4 tétrahydro-1,2,3,6 pyridyl-1, (fluoro-4 phényl)-4 tétrahydro-1,2,3,6 pyridyl-1 ou phényl-4 pipérazinyl-1 dont le noyau phényle est substituée en position -4 par un atome d'halogène ou un radical hydroxy.

Cette réduction s'effectue, de préférence, au moyen d'un borohydrure de métal alcalin tel que le borohydrure de sodium ou le borohydrure de potassium, au sein d'un solvant organique inerte tel qu'un alcool (méthanol, éthanol), le tétrahydrofuranne ou un mélange d'un tel solvant avec l'eau à une température voisine de 20°C.

Les dérivés de formule (III peuvent être obtenus par réaction d'une amine de formules :

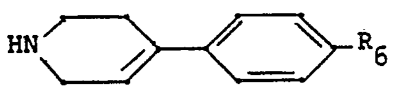

(IV)

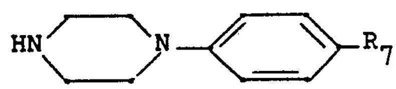

(V)

dans lesquelles $R_6$ représente un atome d'hydrogène ou de fluor et $R_7$ représente un atome d'halogène ou un radical hydroxy avec le dérivé de formule :

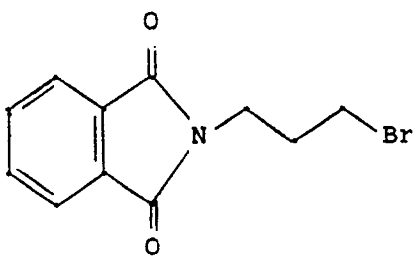

(VI)

Cette réaction s'effectue généralement dans un solvant aromatique inerte tel que le benzène ou le toluène, en présence d'un accepteur d'acide telle qu'une trialkylamine comme la triéthylamine, à une température comprise entre 20°C et la température d'ébullition du solvant.

Le dérivé de formule (VI) peut être préparé par le procédé décrit par T.O. SOINE et BUCHDAHL, Organic Synthesis, 32, 18 (1952).

Le composé de formule (I) pour lequel $R_2$ est un radical hydroxy et $R_1$, $R_3$ sont définis comme précédemment peut aussi être préparé par réaction de (bromo-3 propyl)-2 hydroxy-3 isoindolinone-1 avec une amine de formule (IV) dans laquelle $R_6$ représente un atome de fluor.

Cette réaction s'effectue généralement au sein d'un solvant aromatique inerte tel que le toluène, en présence d'un accepteur d'acide telle qu'une trialkylamine comme la triéthylamine à une température comprise entre 20°C et la température d'ébullition du solvant.

La (bromo-3 propyl)-2 hydroxy-3 isoindolinone-1 peut être préparée par réduction du (bromo-3 propyl)-2 phtalimide de formule (VI).

Cette réduction s'effectue généralement comme décrit précédemment pour la réduction des dérivés de formule (III).

Les composés de formule (I) pour lesquels $R_2$ représente un atome d'hydrogène et $R_1$, $R_3$ sont définis comme précédemment peuvent être obtenus par réduction des dérivés de formule (II) correspondants.

Cette réduction s'effectue, de préférence, au moyen de zinc en présence d'un acide tel que l'acide acétique

à une température voisine de 120°C.

Les composés de formule (I) pour lesquels $R_2$ représente un radical alkyle et $R_1$, $R_3$ sont définis comme précédemment peuvent être préparés par réaction d'un dérivé de formule :

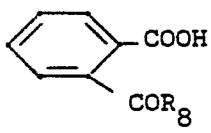
(VII)

dans laquelle $R_8$ représente un radical alkyle avec une amine de formule :

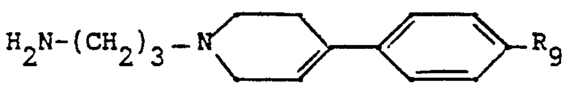
(VIII)

dans laquelle $R_9$ représente un atome d'hydrogène ou de fluor.

Cette réaction s'effectue généralement en présence de cyanoborohydrure de sodium, dans un solvant organique inerte tel que l'acétonitrile à une température voisine de 20°C.

Les amines de formule (VIII) peuvent être obtenues par action d'hydrazine sur un dérivé de formule :

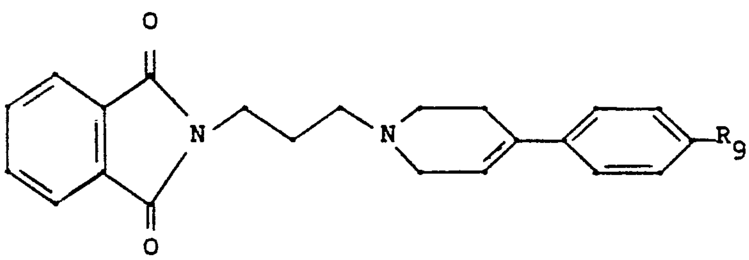
(IX)

dans laquelle $R_9$ a les mêmes significations que dans la formule (VIII).

Cette réaction s'effectue, de préférence, dans un solvant organique tel qu'un alcool (méthanol, éthanol) à une température comprise entre 20°C et la température d'ébullition du solvant.

Les composés de formule (VII) peuvent être obtenus par application ou adaptation de la méthode décrite par E.L. ELIEL et al, J. Am. Chem. Soc., 71, 2251 (1949).

Les composés de formule (I) pour lesquels $R_2$ représente un radical alkylthio et $R_1$, $R_3$ sont définis comme précédemment peuvent être préparés par réaction de formyl-2 benzoate de méthyle avec une amine de formule (VIII) en présence d'un alkylmercaptan.

Cette réaction s'effectue généralement dans un autoclave, au sein d'un solvant organique inerte tel que le tétrahydrofuranne à une température comprise entre 20°C et la température d'ébullition du solvant.

Les composés de formule (I) pour lesquels $R_2$ représente un radical alcoxy, alkylcarbonyloxy ou phénylalkylcarbonyloxy et $R_1$, $R_3$ sont définis comme précédemment peuvent être préparés par réaction des dérivés de formule (II) dans laquelle $R_1$ a les mêmes significations que dans la formule (I) avec un dérivé de formule :

Hal - $R_{10}$ (X)

dans laquelle Hal représente un atome d'halogène (chlore, brome, iode) et $R_{10}$ représente un radical alkyle, alkylcarbonyle ou phénylalkylcarbonyle.

Cette réaction s'effectue généralement en présence d'une base telle que l'hydrure de sodium, dans un solvant organique inerte tel que le tétrahydrofuranne ou le diméthylformamide à une température voisine de 20°C.

Les composés de formule (I) pour lesquels $R_2$ représente un radical phényle, $R_3$ représente un radical hydroxy et $R_1$ est défini comme précédemment peuvent être obtenus par action d'un halogénure de phénylmagnésium tel que le bromure de phénylmagnésium sur un dérivé de formule (III) dans laquelle $R_1$ représente un radical phényl-4 tétrahydro-1,2,3,6 pyridyl-1 ou (fluoro-4 phényl)-4 tétrahydro-1,2,3,6 pyridyl-1.

Cette réaction s'effectue généralement dans un solvant organique inerte tel que le tétrahydrofuranne ou l'éther diéthylique à une température voisine de 20°C.

Les composés de formule (I) pour lesquels R représente un radical phényle ou phénylalkyle, $R_3$ représente un atome d'hydrogène et $R_1$ est défini comme précédemment peuvent être préparés par réduction d'un

dérivé de formule :

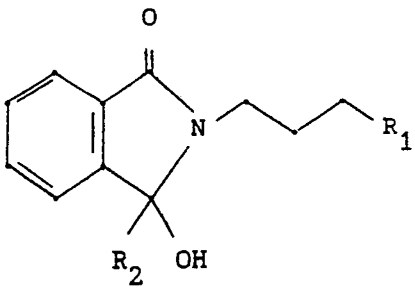

(XI)

dans laquelle $R_2$ représente un radical phényle ou phénylalkyle et $R_1$ est défini comme dans la formule (I).

Cette réduction s'effectue, de préférence, au moyen de cyanoborohydrure de sodium et d'acide trifluoroacétique à une température voisine de 20°C.

Les dérivés de formule (XI) pour lesquels $R_2$ représente un radical phénylalkyle peuvent être obtenus par action d'un halogénure de phénylalkylmagnésium tel qu'un bromure de phénylalkylmagnésium sur un dérivé de formule (III) dans laquelle $R_1$ représente un radical phényl-4 tétrahydro-1,2,3,6 pyridyl-1 ou (fluoro-4 phényl)-4 (tétrahydro-1,2,3,6 pyridyl-1.

Cette réaction s'effectue généralement dans un solvant organique inerte tel que le tétrahydrofuranne ou l'éther diéthylique à une température voisine de 20°C.

Les composés de formule (I) pour lesquels $R_2$ représente un radical $-NR_4R_5$ dans lequel $R_4$ représente un atome d'hydrogène ou un radical alkyle et $R_5$ représente un radical alkyle, phényle, phényle substitué par un atome d'halogène ou pyridyle et $R_1$, $R_3$ sont définis comme précédemment peuvent être préparés par réaction d'une amine de formule :

$HNR_4R_5$ (XII)

dans laquelle $R_4$ et $R_5$ ont les significations mentionnées précédemment sur un dérivé de formule (II) dans laquelle $R_1$ représente un radical phényl-4 tétrahydro-1,2,3,6 pyridyl-1 ou (fluoro-4 phényl)-4 tétrahydro-1,2,3,6 pyridyl-1.

Cette réaction s'effectue généralement au sein d'un solvant aromatique inerte tel que le xylène, en présence d'acide p.toluènesulfonique à la température d'ébullition du solvant.

Les composés de formule (I) pour lesquels $R_2$ représente un radical $-NR_4R_5$ dans lequel $R_4$ et $R_5$ ont les significations mentionnées précédemment peuvent également être préparés par réaction d'un dérivé de formule :

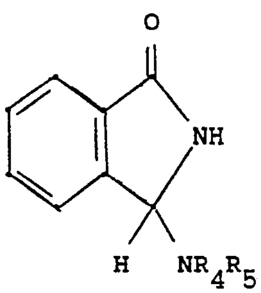

(XIII)

dans laquelle $R_4$ et $R_5$ ont les mêmes significations que dans la formule (I) sur un dérivé de formule :

$Br-(CH_2)_3-R_1$ (XIV)

dans laquelle $R_1$ a les mêmes significations que dans la formule (I).

Cette réaction s'effectue généralement en présence d'un hydrure alcalin tel que l'hydrure de sodium au sein d'un solvant organique inerte tel que le diméthylformamide à une température voisine de 20°C.

Les dérivés de formule (XIII) peuvent être obtenus par action du cyano-2 benzaldéhyde sur une amine $HNR_4R_5$ dans laquelle $R_4$ et $R_5$ ont les mêmes significations que dans la formule (I).

Cette réaction s'effectue généralement au sein d'un solvant organique inerte tel qu'un alcool (méthanol, éthanol) à une température variant de 20°C à la température d'ébullition du solvant.

Les dérivés de formule (XIV) peuvent être obtenus par bromuration des alcools de formule :

$HOCH_2-(CH_2)_2-R_1$ (XV)

dans laquelle $R_1$ a les mêmes significations que dans la formule (XIV).

Cette bromuration s'effectue généralement au moyen d'un agent de bromuration tel que le tribromure de phosphore dans un solvant organique inerte tel que le toluène à une température variant de 20°C à la

température d'ébullition du solvant.

Les dérivés de formule (XV) peuvent être obtenus par action du bromo-3 propanol sur une amine de formules (IV) ou (V).

Cette réaction s'effectue généralement dans un solvant organique inerte tel que le toluène en présence d'un accepteur d'acide tel qu'une trialkylamine comme le triéthylamine à une température variant de 20°C à la température d'ébullition du solvant.

Les mélanges réactionnels obtenus par les divers procédés décrits précédemment sont traités suivant des méthodes classiques physiques (évaporation, extraction, distillation, cristallisation, chromatographie) ou chimiques le cas échéant (formation de sel et régénération de la base ou de l'acide) afin d'isoler les composés de formule (I) à l'état pur.

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés possèdent des propriétés antagonistes de la sérotonine (récepteurs $5HT_2$) et sont donc utiles pour le traitement des affections où la sérotonine est impliquée et notamment les affections du système nerveux central, du système cardiovasculaire et les troubles gastrointestinaux. Ces composés sont, en particulier, utiles pour le traitement de l'anxiété, des troubles du sommeil, de la dépression, des psychoses et notamment de la schizophrénie, de la migraine, de l'asthme, de l'hypertension et de l'urticaire, comme analgésiques et comme inhibiteurs de l'aggrégation plaquettaire.

L'affinité des composés de formule (I) pour les sites récepteurs centraux à sérotonine (type $S_2$) a été déterminée selon une technique inspirée de celle de J.E. LEYSEN et al, Mol. Pharmacol., 21, 301 (1982) qui consiste à mesurer l'affinité des produits pour les sites de liaison de la kétansérine tritiée. Dans ce test, la $CI_{50}$ des composés de formule (I) est inférieure à 10 nM.

Les composés de formule (I) se sont également montrés antagonistes des secousses de la tête (head-twitches) à la mescaline chez la souris selon une technique inspirée de celle de S.J. CORNE et R.W. PICKERING, Psychopharmacologia, 11, 65-78 (1967). Dans ce test, la $DA_{50}$ des composés de formule (I) adminstrés par voie orale est inférieure à 5 mg/kg.

En outre, les composés de formule (I) présentent une toxicité faible. Leur $DL_{50}$ est généralement supérieure à 100 mg/kg par voie orale chez la souris en administration unique.

D'un intérêt particulier sont les composés de formule (I) pour lesquels :

- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un radical alcoxy, hydroxy, alkyle, alkylthio ou alkylcarbonyloxy et $R_3$ représente un atome d'hydrogène
- soit $R_1$ représente un radical (fluoro-4 phényl)-4 pipérazinyl-1, $R_2$ représente un radical alcoxy et $R_3$ représente un atome d'hydrogène ;

étant entendu que lorsque $R_1$ représente le radical phényl-4 tétrahydro-1,2,3,6 pyridyl-1, $R_2$ n'est pas le radical hydroxy.

Parmi ces composés, plus spécialement actifs, sont les composés de formule (I) pour lesquels $R_2$ représente un radical alcoxy.

Sont notamment remarquables les composés suivants :

- méthoxy-3 [((fluoro-4 phényl)-4 tétrahydro-1,2,3,6 pyridyl-1)-3 propyl]-2 isoindolinone-1
- méthoxy-3 [((fluoro-4 phényl)-4 pipérazinyl-1)-3 propyl]-2 isoindolinone-1
- méthoxy-3 [(phényl-4 tétrahydro-1,2,3,6 pyridyl-1)-3 propyl]-2 isoindolinone-1
- éthoxy-3 [(phényl-4 tétrahydro-1,2,3,6 pyridyl-1)-3 propyl]-2 isoindolinone-1
- méthylthio-3 [(phényl-4 tétrahydro-1,2,3,6 pyridyl-1)-3 propyl]-2 isoindolinone-1
- méthyl-3 [(phényl-4 tétrahydro-1,2,3,6 pyridyl-1)-3 propyl]-2 isoindolinone-1
- acétoxy-3 [(phényl-4 tétrahydro-1,2,3,6 pyridyl-1)-3 propyl]-2 isoindolinone-1
- hydroxy-3 [((fluoro-4 phényl)-4 tétrahydro-1,2,3,6 pyridyl-1)-3 propyl]-2 isoindolinone-1

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables peuvent être cités les sels d'addition avec les acides minéraux tels que chlorhydrate, sulfate, nitrate phosphate ou organiques tels que acétate, propionate, oxalate, succinate, benzoate, fumarate, maléate, méthanesulfonate, iséthionate, théophyllineacétate, salicylate, phénolphtalinate, méthylène-bis-β-oxynaphtoate ou des dérivés de substitution de ces composés.

Les exemples suivants, donnés à titre non limitatif, montrent comment l'invention peut être mise en pratique.

EXEMPLE 1

A une solution de 18,9 g de (bromo-3 propyl)-2 hydroxy-3 isoindolinone-1 dans 200 cm3 de toluène, on ajoute, à une température voisine de 20°C et en 10 minutes, une solution de 12,3 g de (fluoro-4 phényl)-4 tétrahydro-1,2,3,6 pyridine en solution dans 20 cm3 de toluène, puis une solution de 9,8 cm3 de triéthylamine dans 20 cm3 de toluène. On agite à une température voisine de 111°C pendant 3 heures. Aprés refroidissement à une température voisine de 20°C, le précipité formé est séparé par filtration et lavé avec 3 fois 50 cm3 de toluène. Les phases organiques sont réunies et concentrées à sec sous pression réduite (20

mm de mercure ; 2,7 kPa) à 60° C. Le résidu obtenu est dissous dans 50 cm3 de chlorure de méthylène et la solution est versée sur 1 kg de silice contenue dans une colonne de 8 cm de diamètre. On élue avec un mélange de chlorure de méthylène et de méthanol (98-2 en volumes). Les premiers 4 000 cm3 sont éliminés et les 4 000 cm3 suivants sont concentrés à sec sous pression réduite (20 mm de mercure ; 2,7 kPa) à 40° C. L'huile obtenue est dissoute dans 70 cm3 de méthyléthylcétone. On ajoute une solution de 1,6 g d'acide oxalique dans 20 cm3 de méthyléthylcétone et maintient l'agitation pendant environ 1 heure. Le précipité formé est séparé par filtration, lavé avec 20 cm3 de méthyléthylcétone puis recristallisé dans 150 cm3 d'un mélange d'isopropanol et d'oxyde isopropylique (75-25 en volumes) bouillant. On obtient ainsi 4,8 g d'oxalate d'hydroxy-3 [((fluoro-4 phényl)-4 tétrahydro-1,2,3,6 pyridyl-1)-3 propyl]-2 isoindolinone -1 fondant à 93° C.

La (bromo-3 propyl)-2 hydroxy-3 isoindolinone-1 peut être préparée de la façon suivante : à une solution de 53,6 g de (bromo-3 propyl)-2 phtalimide dans 450 cm3 de méthanol, on ajoute 21,6 g de borohydrure de potassium, à une température voisine de 20° C et poursuit l'agitation pendant 24 heures. La solution obtenue est versée sur 1 000 cm3 d'eau distillée et extraite avec 4 fois 150 cm3 d'acétate d'éthyle. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 100 cm3 de chlorure de méthylène et la solution est versée sur 750 g de silice contenue dans une colonne de 8 cm de diamètre. On élue avec 2 litres de chlorure de méthylène puis avec 1 litre d'un mélange de chlorure de méthylène et de méthanol (99-1 en volumes), 1 litre d'un mélange de chlorure de méthylène et de méthanol (98-2 en volumes) puis 1 litre d'un mélange de chlorure de méthylène et de méthanol (97-3 en volumes). Les éluats correspondants sont éliminés.On élue ensuite avec 3 litres d'un mélange de chlorure de méthylène et de méthanol (97-3 en volumes) et l'éluat correspondant est concentré à sec sous pression réduite (20 mm de mercure ; 2,7 kPa) à 50° C. On obtient 18,9 g d'une huile blanche dont le Rf sur plaque de silice et dans un mélange de chlorure de méthylène et de méthanol (95-5 en volumes) est de 0,39.

Le (bromo-3 propyl)-2 phtalimide peut être préparé selon la méthode décrite par T.O. SOINE et BUCHDAHL, Organic Synthesis, 32, 18 (1952).

EXEMPLE 2

A une solution agitée de 5,7 g de [((fluoro-4 phényl)-4 tétrahydro-1,2,3,6 pyridyl-1)-3 propyl]-2 hydroxy-3 isoindolinone-1 dans 145 cm3 de méthanol, on ajoute, à une température voisine de 20° C et en 10 minutes, 28,5 cm3 d'acide sulfurique concentré. On poursuit l'agitation pendant 5 heures à une température voisine de 65° C. Après refroidissement de la solution à une température voisine de 0° C, on ajoute, en 1 heure, 70 cm3 d'une solution d'ammoniaque aqueux à 33 %. Le précipité formé est filtré et lavé avec 50 cm3 de méthanol. On dilue le filtrat avec 200 cm3 d'eau distillée et 50 cm3 d'une solution d'ammoniaque aqueux à 33 % et extrait avec 3 fois 20 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 10 cm3 de chlorure de méthylène et la solution est versée sur 550 g de silice contenue dans une colonne de 8 cm de diamètre. On élue avec un mélange de chlorure de méthylène et de méthanol (90-10 en volumes). Les 900 premiers cm3 sont éliminés et les 200 cm3 suivants sont évaporés à sec sous pression réduite (20 mm de mercure ; 2,7 kPa) à 50° C. Le résidu obtenu est dissous dans 10 cm3 de méthyléthylcétone. On ajoute une solution de 0,6 g d'acide oxalique dans 5 cm3 de méthyléthylcétone et poursuit l'agitation pendant 1 heure à une température voisine de 20° C. Le précipité formé est séparé par filtration. On obtient ainsi 2,4 g d'oxalate de méthoxy-3 [((fluoro-4 phényl)-4 tétrahydro-1,2,3,6 pyridyl-1)-3 propyl]-2 isoindolinone-1 fondant à 139° C.

EXEMPLE 3

A une suspension de 0,8 g d'hydrure de sodium (à 50 % en suspension dans l'huile) dans 10 cm3 de diméthylformamide anhydre, on ajoute, à une température voisine de 20° C et en 15 minutes, une solution de 5,2 g de [(phényl-4 tétrahydro-1,2,3,6 pyridyl-1)-3 propyl]-2 hydroxy-3 isoindolinone-1 dans 50 cm3 de diméthylformamide anhydre et poursuit l'agitation pendant 2 heures. On ajoute ensuite, en 5 minutes, 1 cm3 d'iodure de méthyle et continue l'agitation pendant encore 20 heures. La suspension obtenue est versée sur 1 000 cm3 d'eau distillée et extraite avec 3 fois 1 000 cm3 de chlorure de méthylène. Les extraits organiques sont réunis, séchés sur sulfate de magnésium anhydre, filtrés et concentrés à sec sous pression réduite (20 mm de mercure; 2,7 kPa) à 40° C. Le résidu obtenu est dissous dans 20 cm3 de chlorure de méthylène et la solution est versée sur 300 g de silice contenue dans une colonne de 7 cm de diamètre. On élue avec 1 litre d'un mélange de chlorure de de méthylène et de méthanol (98-2 en volumes) et l'éluat correspondant est évaporé à sec sous pression réduite (20 mm de mercure ; 2,7 kPa) à 50° C. Le résidu obtenu est dissous dans 30 cm3 de méthyléthylcétone. On ajoute une solution de 0,7 g d'acide oxalique dans 10 cm3 de méthyléthylcétone et poursuit l'agitation pendant 1 heure à une température voisine de 20° C. Le précipité formé est séparé par filtration. On obtient ainsi, 2,6 g d'oxalate de méthoxy-3 [(phényl-4 tétrahydro-1,2,3,6 pyridyl-1)-3 propyl]-2 isoindolinone-1 fondant à 160° C.

La [(phényl-4 tétrahydro-1,2,3,6 pyridyl-1)-3 propyl]-2 hydroxy-3 isoindolinone-1 peut être obtenue de la manière suivante : à une solution de 21 g de [(phényl-4 tétrahydro-1,2,3,6 pyridyl-1)-3 propyl]-2 phtalimide dans 210 cm3 de méthanol, on ajoute 3,24 g de borohydrure de potassium, à une température voisine de 20° C et poursuit l'agitation pendant 24 heures. On ajoute à nouveau 1,62 g de borohydrure de potassium et continue l'agitation pendant encore 6 heures. La solution obtenue est ensuite versée sur 1 000 cm3 d'eau

distillée et extraite avec 4 fois 150 cm3 d'acétate d'éthyle. Les extraites organiques sont réunis, séchés sur sulfate de magnésium anhydre, filtrés et concentrés à sec sous pression réduite (20 mm de mercure ; 2,7 kPa) à 40°C. Le résidu obtenu est dissous dans 150 cm3 d'acétonitrile. On ajoute une solution de 5,4 g d'acide oxalique dans 200 cm3 d'acétonitrile et poursuit l'agitation pendant 1 heure à une température voisine de 20°C. Le précipité formé est séparé par filtration, puis recristallisé dans 700 cm3 d'acétonitrile bouillant. On obtient ainsi 10,2 g d'oxalate de [(phényl-4 tétrahydro-1,2,3,6 pyridyl-1)-3 propyl]-2 hydroxy-3 isoindolinone-1 fondant à 130-135°C.

Le [(phényl-4 tétrahydro-1,2,3,6 pyridyl-1)-3 propyl]-2 phtalimide peut être préparé de la manière suivante : à une solution agitée de 16,1 g de (bromo-3 propyl)-2 phtalimide et de 10,4 g de phényl-4 tétrahydro-1,2,3,6 pyridine dans 160 ml de toluène, on ajoute, en 10 minutes et à une température voisine de 20°C, 7,4 cm3 de triéthylamine. On poursuit l'agitation pendant 6 heures à une température voisine de 111°C. Après refroidissement à une température voisine de 20°C, le précipité formé est séparé par filtration et lavé avec 30 cm3 de toluène. Les phases organiques sont réunies et concentrées à sec sous pression réduite (20 mm de mercure ; 2,7 kPa) à 60°C. Le précipité obtenu est recristallisé dans 65 cm3 d'isopropanol bouillant. On obtient ainsi 21 g de [(phényl-4 tétrahydro-1,2,3,6 pyridyl-1)-3 propyl]-2 phtalimide fondant à 90°C.

Le (bromo-3 propyl)-2-phtalimide peut être préparé suivant la méthode décrite par T.O. SOINE et BUCHDAHL, Organic Synthesis, 32,18 (1952).

EXEMPLE 4

On opère comme à l'exemple 2, à partir d'une solution agitée de 6,8 g de [((fluoro-4 phényl)-4 pipérazinyl-1)-3 propyl]-2 hydroxy-3 isoindolinone-1 dans 170 cm3 de méthanol à laquelle on ajoute, à une température voisine de 20°C et en 15 minutes, 33,5 cm3 d'acide sulfurique concentré. On poursuit l'agitation pendant 5 heures à une température voisine de 65°C. Après refroidissement de la solution à une température voisine de 0°C, on ajoute, en 1 heure, 83 cm3 d'une solution d'ammoniaque aqueux à 33 %. Le précipité formé est filtré et lavé avec 50 cm3 de méthanol. On dilue le filtrat avec 200 cm3 d'eau distillée et 50 cm3 d'une solution d'ammoniaque aqueux à 33 % et extrait avec 3 fois 200 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40°C. Le résidu obtenu est dissous dans 20 cm3 de chlorure de méthylène et la solution est versée sur 500 g de silice contenue dans une colonne de 8 cm de diamètre. On élue avec un mélange de chlorure de méthylène et de méthanol (99-1 en volumes). Les 300 premiers cm3 sont éliminés et les 3 500 cm3 suivants sont évaporés à sec sous pression réduite (20 mm de mercure ; 2,7 kPa) à 50°C. Le résidu obtenu est dissous dans 50 cm3 d'acétonitrile. On ajoute une solution de 1,1 g d'acide oxalique dans 25 cm3 d'acétonitrile et poursuit l'agitation pendant 1 heure à une température voisine de 20°C. Le précipité formé est séparé par filtration. On obtient ainsi 4,1 g d'oxalate de méthoxy-3 [((fluoro-4 phényl)-4 pipérazinyl-1)-3 propyl]-2 isoindolinone-1 fondant à 177°C.

La [((fluoro-4 phényl)-4 pipérazinyl-1)-3 propyl]-2 hydroxy-3 isoindolinone-1 peut être préparée comme à l'exemple 3 pour la préparation de la [(phényl-4 tétrahydro-1,2,3,6 pyridyl-1)-3 propyl]-2 hydroxy-3 isoindolinone-1, mais à partir d'une solution agitée de 10,3 g de [((fluoro-4 phényl)-4 pipérazinyl-1)-3 propyl]-2 phtalimide dans 200 cm3 de méthanol et 10 cm3 d'eau distillée dans laquelle on ajoute, à une température voisine de 20°C, 1,51 g de borohydrure de potassium. On poursuit l'agitation pendant 24 heures. La solution obtenue est versée sur 130 cm3 d'eau distillée et extraite avec 4 fois 100 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40°C. Le résidu obtenu est dissous dans 125 cm3 d'acétonitrile bouillant. Après refroidissement à une température voisine de 20°C, le précipité formé est séparé par filtration. On obtient ainsi 6,9 g de [((fluoro-4 phényl)-4 pipérazinyl-1)-3 propyl]-2 hydroxy-3 isoindolinone-1 fondant à 155°C.

Le [((fluoro-4 phényl)-4 pipérazinyl-1)-3 propyl]-2 phtalimide peut être préparé comme à l'exemple 3 pour la préparation de la [phényl-4 tétrahydro-1,2,3,6 pyridyl-1)-3 propyl]-2 phtalimide, mais à partir d'une solution agitée de 15 g de (bromo-3 propyl)-2 phtalimide et de 10,1 g de (fluoro-4 phényl)-4 pipérazine dans 500 cm3 de toluène à laquelle on ajoute, en 10 minutes, et à une température voisine de 20°C, 7,8 cm3 de triéthylamine. Le précipité obtenu est dissous dans 50 cm3 de chlorure de méthylène et versé sur 1 250 g de silice contenue dans une colonne de 8 cm de diamètre. On élue avec un mélange de chlorure de méthylène et de méthanol (98-2 en volumes). Les 750 premiers cm3 sont éliminés et les éluats correspondants aux 2 250 cm3 suivants sont concentrés à sec sous pression réduite (20 mm de mercure ; 2,7 kPa) à 50°C. On obtient 13,3 g d'une huile orangée dont le Rf sur plaque de silice et dans un mélange de chlorure de méthylène et de méthanol (95-5 en volumes) est de 0,5.

EXEMPLE 5

On opère comme à l'exemple 2, mais à partir d'une solution agitée de 4,8 g de [((chloro-4 phényl)-4 pipérazinyl-1)-3 propyl]-2 hydroxy-3 isoindolinone-1 dans 115 cm3 de méthanol à laquelle on ajoute, à une température voisine de 20°C et en 10 minutes 22,6 cm3 d'acide sulfurique concentré. On poursuit l'agitation pendant 5 heures à une température voisine de 65°C. Après refroidissement de la solution à une température voisine de 0°C, on ajoute, en 1 heure, 55 cm3 d'une solution d'ammoniaque aqueux à 33 %. Le précipité formé est filtré et lavé avec 50 cm3 de méthanol. On dilue le filtrat avec 200 cm3 d'eau distillée et 50 cm3 d'une solution d'ammoniaque aqueux à 33 % et extrait avec 3 fois 200 cm3 de chlorure de méthylène. Les extraits

organiques sont réunis, séchés sur sulfate de magnésium anhydre, filtrés et concentrés à sec sous pression réduite (20 mm de mercure ; 2,7 kPa) à 40° C. Le résidu obtenu est dissous dans 20 cm3 de chlorure de méthylène et la solution est versée sur 500 g de silice contenue dans une colonne de 8 cm de diamètre. On élue avec un mélange de chlorure de méthylène et de méthanol (98,5-1,5 en volumes). Les 100 premiers cm3 sont éliminés et les 630 cm3 suivants sont évaporés à sec sous pression réduite (20 mm de mercure ; 2,7 kPa) à 50° C. Le résidu obtenu est dissous dans 30 cm3 d'acétonitrile. On ajoute une solution de 0,63 g d'acide oxalique dans 15 cm3 d'acétonitrile et poursuit l'agitation pendant 1 heure à une température voisine de 20° C. Le précipité formé est séparé par filtration. On obtient ainsi 2,9 g d'oxalate de méthoxy-3 [((chloro-4 phényl)-4 pipérazinyl-1)-3 propyl]-2 isoindolinone-1 fondant à 200° C.

La [((chloro-4 phényl)-4 pipérazinyl-1)-3 propyl]-2 hydroxy-3 isoindolinone-1 peut être préparée comme à l'exemple 3 pour la préparation de la [(phényl-4 tétrahydro-1,2,3,6 pyridyl-1)-3 propyl]-2 hydroxy-3 isoindolinone-1, mais à partir d'une solution agitée de 4 g de [((chloro-4 phényl)-4 pipérazinyl-1)-3 propyl]-2 phtalimide dans 20 cm3 de méthanol et 10 cm3 d'eau distillée à laquelle on ajoute, à une température voisine de 20° C, 0,56 g de borohydrure de potassium. On poursuit l'agitation pendant 24 heures. La solution obtenue est versée sur 50 cm3 d'eau distillée et extraite avec 4 fois 50 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 30 cm3 d'acétonitrile bouillant. Après refroidissement à une température voisine de 20° C, le précipité formé est séparé par filtration. On obtient ainsi 2,4 g de [((chloro-4 phényl)-4 pipérazinyl-1)-3 propyl]-2 hydroxy-3 isoindolinone-1 fondant à 173° C.

Le [((chloro-4 phényl)-4 pipérazinyl-1)-3 propyl]-2 phtalimide peut être préparé comme à l'exemple 3 pour la préparation de la [(phényl-4 tétrahydro-1,2,3,6 pyridyl-1)-3 propyl]-2 hydroxy-3 isoindolinone-1, mais à partir d'une solution agitée de 5,3 g de (bromo-3 propyl)-2 phtalimide et de 5,4 g de (chloro-4 phényl)-4 pipérazine dans 100 cm3 de toluène à laquelle on ajoute, en 5 minutes et à une température voisine de 20° C, 2,8 cm3 de triéthylamine. Le précipité obtenu est dissous dans 20 cm3 de chlorure de méthylène puis versé sur 200 g de silice contenue dans une colonne de 4 cm de diamètre. On élue avec un mélange de chlorure de méthylène et de méthanol (98-2 en volumes). Les 300 premiers cm3 sont éliminés et les éluats correspondants aux 400 cm3 suivants sont concentrés à sec sous pression réduite (20 mm de mercure ; 2,7 kPa) à 50° C. On obtient ainsi 4 g de [((chloro-4 phényl)-4 pipérazinyl-1)-3 propyl]-2 phtalimide fondant à 120° C.

EXEMPLE 6

On opère comme à l'exemple 2, mais à partir d'une solution agitée de 7 g de [(phényl-4 tétrahydro-1,2,3,6 pyridyl-1)-3 propyl]-2 hydroxy-3 isoindolinone-1 dans 265 cm3 d'éthanol à laquelle on ajoute, à une température voisine de 20° C et en 15 minutes, 37 cm3 d'acide sulfurique concentré. On poursuit l'agitation pendant 4 heures à une température voisine de 65° C. Après refroidissement de la solution à une température voisine de 0° C, on ajoute, en 1 heure, 90 cm3 d'une solution d'ammoniaque aqueux à 33 %. Le précipité formé est filtré, lavé avec 50 cm3 de méthanol. On dilue le filtrat avec 200 cm3 d'eau distillée et 70 cm3 d'une solution d'ammoniaque aqueux à 33 % et extrait avec 3 fois 250 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 30 cm3 de chlorure de méthylène et la solution est versée sur 500 g de silice contenue dans une colonne de 8 cm de diamètre. On élue avec un mélange de chlorure de méthylène et de méthanol (95-5 en volumes). Les 1 000 premiers cm3 sont éliminés, les 900 cm3 suivants sont évaporés à sec sous pression réduite (20 mm de mercure ; 2,7 kPa) à 50° C. Le résidu obtenu est dissous dans 50 cm3 de méthyléthylcétone. On ajoute une solution de 1,2 g d'acide oxalique dans 20 cm3 de méthyléthylcétone et poursuit l'agitation pendant 1 heure à une température voisine de 20° C. Le précipité formé est séparé par filtration. On obtient ainsi, 4,9 g d'oxalate d'éthoxy-3 [(phényl-4 tétrahydro-1,2,3,6 pyridyl-1)-3 propyl]-2 isoindolinone-1 fondant à 150° C.

EXEMPLE 7

A une solution agitée, dans un appareil de Dean Stark, de 5 g de [(phényl-4 tétrahydro-1,2,3,6 pyridyl-1)-3 propyl]-2 hydroxy-3 isoindolinone-1 et de 1,7 g d'aniline dans 100 cm3 de xylène, on ajoute, à une température voisine de 20° C, 50 mg d'acide para-toluènesulfonique. On poursuit l'agitation pendant 9 heures à une température voisine de 135° C. Après refroidissement à une température voisine de 20° C, la solution est lavée avec 2 fois 50 cm3 d'une solution de bicarbonate de sodium aqueux puis avec 2 fois 30 cm3 d'eau distillée. Les extraits organiques sont réunis, séchés sur sulfate de magnésium anhydre, filtrés et concentrés à sec sous pression réduite (20 mm de mercure ; 2,7 kPa) à 80° C. Le résidu obtenu est dissous dans 10 cm3 de chlorure de méthylène et versé sur 100 g de silice contenue dans une colonne de 2 cm de diamètre. On élue avec 1 300 cm3 d'un mélange de chlorure de méthylène et de méthanol (95-5 en volumes) et l'éluat correspondant est concentré à sec sous pression réduite (20 mm de mercure ; 2,7 kPa). le précipité formé est recristallisé dans 30 cm3 d'acétonitrile bouillant. On obtient 5,7 g de phénylamino-3 [(phényl-4 tétrahydro-1,2,3,6 pyridyl-1)-3 propyl]-2 isoindolinone-1 fondant à 119° C.

EXEMPLE 8

A une solution agitée, dans un appareil de Dean Stark, de 10 g de [(phényl-4 tétrahydro-1,2,3,6 pyridyl-1)-3 propyl]-2 hydroxy-3 isoindolinone-1 et de 4,6 g de chloro-4-phénylamine dans 230 cm3 de xylène, on ajoute, à

une température voisine de 20° C, 50 mg d'acide para-toluènesulfonique. On poursuit l'agitation pendant 6 heures à une température voisine de 135° C. Après refroidissement à une température voisine de 20° C, le précipité formé est filtré, lavé avec 2 fois 50 cm3 de xylène, séché sous pression réduite (20 mm de mercure ; 2,7 kPa) et recristallisé dans 360 cm3 d'acétonitrile bouillant. On obtient 7,1 g de (chloro-4 phénylamino)-3 [(phényl-4 tétrahydro-1,2,3,6 pyridyl-1)-3 propyl]-2 isoindolinone-1 fondant à 188° C.

EXEMPLE 9

A une solution agitée, dans un appareil de Dean Stark, de 6 g de [(phényl-4 tétrahydro-1,2,3,6 pyridyl)-3 propyl]-2 hydroxy-3 isoindolinone-1 et de 2 g d'amino-2 pyridine dans 150 cm3 de xylène, on ajoute, à une température voisine de 20° C, 50 mg d'acide para-toluènesulfonique. On poursuit l'agitation pendant 5 heures à une température voisine de 135° C. Après refroidissement à une température voisine de 20° C, la solution est lavée avec 2 fois 100 cm3 d'une solution de bicarbonate de sodium aqueux puis avec 2 fois 50 cm3 d'eau distillée. Les extraits organiques sont réunis, séchés sur sulfate de magnésium anhydre, filtrés et concentrés à sec sous pression réduite (20 mm de mercure ; 2,7 kPa) à 80° C. Le précipité est recristallisé dans 45 cm3 d'acétonitrile bouillant. On obtient 3,7 g de (pyridyl-2 amino)-3 [(phényl-4 tétrahydro-1,2,3,6 pyridyl-1)-3 propyl]-2-isoindolinone-1 fondant à 153° C.

EXEMPLE 10

A une suspension de 2,2 g d'hydrure de sodium (à 50 % en suspension dans l'huile) dans 100 cm3 de diméthylformamide anhydre, on ajoute, à une température voisine de 20° C et en 30 minutes une solution de 10 g de N-méthyl N-phénylamino-3 isoindolinone-1 dans 200 cm3 de diméthylformamide anhydre et poursuit l'agitation pendant 1 heure. On ajoute ensuite, en 15 minutes, 13 g de (phényl-4 tétrahydro-1,2,3,6, pyridyl-1)-3 bromo-1 propane en solution dans 100 cm3 de diméthylformamide et continue l'agitation pendant encore 20 heures. La solution obtenue est évaporée à sec sous pression réduite (1 mm de mercure ; 0,1 kPa). Le résidu obtenu est dissous dans 200 cm3 de chlorure de méthylène et lavé avec 3 fois 50 cm3 d'eau distillée. Les extraits organiques sont séchés sur sulfate de magnésium anhydre, filtrés et concentrés à sec sous pression réduite (20 mm de mercure ; 2,7 kPa) à 40° C. Le résidu obtenu est dissous dans 40 cm3 de chlorure de méthylène puis versé sur 800 g de silice contenue dans une colonne de 8 cm de diamètre. On élue avec un mélange de chlorure de méthylène et de méthanol (95-5 en volumes). Les 2 300 premiers cm3 sont éliminés et les 1 100 cm3 suivants sont concentrés à sec sous pression réduite (20 mm de mercure ; 2,7 kPa). Le résidu obtenu est dissous dans 50 cm3 de méthyléthylcétone. On ajoute une solution de 1,1 g d'acide oxalique dans 15 cm3 de méthyléthylcétone et poursuit l'agitation pendant 1 heure à une température voisine de 20° C. Le précipité formé est séparé par filtration. On obtient ainsi 5,4 g d'oxalate de N-méthyl N-phénylamino-3 [(phényl-4 tétrahydro-1,2,3,6, pyridyl-1)-3 propyl]-2 isoindolinone-1 fondant à 159° C.

La N-méthyl N-phénylamino-3 isoindolinone-1 peut être préparée de la manière suivante : à une solution de 26 g de cyano-2 benzaldéhyde dans 280 cm3 de méthanol, on ajoute, en 10 minutes et à une température voisine de 20° C, 65 g de N-méthyl N-phénylamine. On porte le mélange réactionnel à une température voisine de 40° C pendant 24 heures. Après refroidissement à une température voisine de 20° C, la solution est concentrée à sec sous pression réduite (20 mm de mercure ; 2,7 kPa) à 50° C. Le résidu obtenu est dissous dans 50 cm3 de chlorure de méthylène et versé sur 600 cm3 de silice contenue dans une colonne de 8 cm de diamètre. On élue avec un mélange de chlorure de méthylène et de méthanol (98-2 en volumes). Les 2 000 premiers cm3 sont éliminés et l'éluat correspondant aux 2 000 cm3 suivants est concentré à sec sous pression réduite (20 mm de mercure ; 2,7 kPa). On obtient une huile brune dont le Rf sur plaque de silice et dans un mélange de chlorure de méthylène et de méthanol (97-3 en volumes) est de 0,4.

Le (phényl-4 tétrahydro-1,2,3,6 pyridyl-1)-3 bromo-1 propane peut être préparé de la manière suivante : à une solution fortement agitée de 8,7 g de (phényl-4 tétrahydro-1,2,3,6 pyridyl-1)-3 propanol-1 dans 100 cm3 de toluène, on ajoute, en 5 minutes et à une température voisine de 20° C, 2,7 cm3 de tribromure de phosphore. On porte la suspension à une température voisine de 111° C pendant 2 heures. Après refroidissement à une température voisine de 20° C, la suspension est filtrée, le précipité obtenu est dissous dans 250 cm3 de chlorure de méthylène. La phase organique est lavée avec 150 cm3 d'eau distillée, séchée sur sulfate de magnésium anhydre puis concentrée à sec sous pression réduite (20 mm de mercure ; 2,7 kPa). On obtient ainsi 14 g de bromhydrate de (phényl-4 tétrahydro-1,2,3,6 pyridyl-1)-3 bromo-1 propane fondant à 185° C.

Le (phényl-4 tétrahydro-1,2,3,6 pyridyl-1)-3 propanol-1 peut être préparé de la manière suivante : à une solution de 25 cm3 de bromo-3 propanol-1 et de 42 g de phényl-4 tétrahydro-1,2,3,6 pyridine dans 500 cm3 de toluène, on ajoute, en 10 minutes et à une température voisine de 20° C, 36,7 cm3 de triéthylamine. On porte la solution à une température voisine de 111° C pendant 16 heures. Après refroidissement à une température voisine de 20° C, la suspension est filtrée, le précipité est lavé avec 2 fois 50 cm3 de toluène. Les extraits organiques réunis sont traités au noir animal et évaporés à sec sous pression réduite (20 mm de mercure ; 2,7 kPa) à 60° C. On obtient ainsi 42 g d'une huile jaune dont le Rf sur plaque de silice de dans un mélange de chlorure de méthylène et de méthanol (90-10 en volumes) est de 0,25.

EXEMPLE 11

A une suspension de 3,5 g d'hydrure de sodium (à 50 % en suspension dans l'huile) dans 150 cm3 de diméthylformamide anhydre, on ajoute, à une température voisine de 20° C et en 30 minutes, une solution de 11,7 g de diméthylamino-3 isoindolinone-1 dans 300 cm3 de diméthylformamide anhydre et poursuit l'agitation

pendant 1 heure. On ajoute ensuite, en 15 minutes, 20,5 g de (phényl-4 tétrahydro-1,2,3,6 pyridyl-1)-3 bromo-1 propane en solution dans 150 cm3 de diméthylformamide et continue l'agitation pendant encore 20 heures. La solution obtenue est évaporée à sec sous pression réduite (1 mm de mercure ; 0,1 kPa). Le résidu obtenu est dissous dans 350 cm3 de chlorure de méthylène et lavé avec 3 fois 150 cm3 d'eau distillée. L'extrait organique est séché sur sulfate de magnésium anhydre, filtré et concentré à sec sous pression réduite (20 mm de mercure ; 2,7 kPa) à 40° C. Le résidu obtenu est dissous dans 50 cm3 de chlorure de méthylène et versé sur 1 000 g de silice contenue dans une colonne de 8 cm de diamètre. On élue avec un mélange de chlorure de méthylène et de méthanol (95-5 en volumes). Les 2 300 premiers cm3 sont éliminés et l'éluat correspondant aux 1 500 cm3 suivants est concentré à sec sous pression réduite (20 mm de mercure ; 2,7 kPa). Le résidu obtenu est dissous dans 80 cm3 de méthyléthylcétone. On ajoute une solution de 1,9 g d'acide oxalique dans 20 cm3 de méthyléthylcétone et poursuit l'agitation pendant 1 heure à une température voisine de 20° C. Le précipité formé est séparé par filtration. On obtient ainsi 7,6 g d'oxalate de diméthylamino-3 [(phényl-4 tétrahydro-1,2,3,6 pyridyl-1)-3 propyl]-2 isoindolinone-1 fondant à 159° C.

La diméthylamino-3 isoindolinone-1 peut être préparée de la manière suivante : à une solution de 40 g de cyano-2 benzaldéhyde dans 450 cm3 de méthanol, on ajoute, en 10 minutes et à une température voisine de 20° C, 60 cm3 de diméthylamine. On porte le mélange réactionnel à une température voisine de 40° C pendant 24 heures. Aprés refroidissement à une température voisine de 20° C, la solution est concentrée à sec sous pression réduite (20 mm de mercure ; 2,7 kPa) à 50° C. Le résidu obtenu est dissous dans 300 cm3 d'acétonitrile bouillant, traité au noir animal et filtré à chaud. Après refroidissement à une température voisine de 20° C, le précipité formé est filtré. On obtient ainsi 10,1 g de diméthylamino-3 isoindolinone-1 dont le Rf sur plaque de silice et dans un mélange de chlorure de méthylène et de méthanol (90-10 en volumes) est de 0,35.

EXEMPLE 12

A une suspension de 2,2 g d'hydrure de sodium (à 50 % en suspension dans l'huile) dans 100 cm3 de diméthylformamide anhydre, on ajoute, à une température voisine de 20° C et en 15 minutes, une solution de 6,8 g de méthylamino-3 isoindolinone-1 dans 200 cm3 de diméthylformamide anhydre et poursuit l'agitation pendant 1 heure. On ajoute ensuite, en 10 minutes, 13 g de (phényl-4 tétrahydro-1,2,3,6 pyridyl-1)-3 bromo-1 propane en solution dans 100 cm3 de diméthylformamide et continue l'agitation pendant encore 20 heures. La solution obtenue est évaporée à sec sous pression réduite (1 mm de mercure ; 0,1 kPa). Le résidu obtenu est dissous dans 500 cm3 de chlorure de méthylène et lavé avec 3 fois 500 cm3 d'eau distillée. La phase organique est séchée sur sulfate de magnésium anhydre, filtrée et concentrée à sec sous pression réduite (20 mm de mercure ; 2,7 kPa) à 40° C. Le résidu obtenu est dissous dans 20 cm3 de chlorure de méthylène et versé sur 750 g de silice contenue dans une colonne de 8 cm de diamètre. On élue avec un mélange de chlorure de méthylène et de méthanol (92-8 en volumes). Les 3 750 premiers cm3 sont éliminés et l'éluat correspondant aux 600 cm3 suivants est concentré à sec sous pression réduite (20 mm de mercure ; 2,7 kPa). Le résidu obtenu est dissous dans 50 cm3 de méthyléthylcétone. On ajoute une solution de 1,2 g d'acide oxalique dans 20 cm3 de méthyléthylcétone et poursuit l'agitation pendant 1 heure à une température voisine de 20° C. Le précipité formé est séparé par filtration. On obtient ainsi 5 g d'oxalate de méthylamino-3 [(phényl-4 tétrahydro-1,2,3,6 pyridyl-1)-3 propyl]-2 isoindolinone-1 fondant à 122° C.

La méthylamino-3 isoindolinone-1 peut être préparée de la manière suivante : dans un autoclave, on ajoute à une solution de 26 g de cyano-2 benzaldéhyde dans 300 cm3 de méthanol, en 10 minutes et à une température voisine de -70° C, 18,6 g de diméthylamine. On porte ensuite le milieu réactionnel à une température voisine de 40° C pendant 24 heures. Après refroidissement à une température voisine de 20° C, la solution est concentrée à sec sous pression réduite (20 mm de mercure ; 2,7 kPa) à 50° C. Le précipté obtenu est recristallisé dans 150 cm3 d'acétonitrile bouillant. On obtient ainsi 20,9 g de méthylamino-3 isoindolinone-1 fondant à 152° C.

EXEMPLE 13

A une suspension de 1,2 g d'hydrure de sodium (à 50 % en suspension dans l'huile) dans 15 cm3 de diméthylformamide anhydre, on ajoute, à une température voisine de 20° C et en 15 minutes, une solution de 8 g de [(phényl-4 tétrahydro-1,2,3,6 pyridyl-1)-3 propyl]-2 hydroxy-3 isoindolinone-1 dans 75 cm3 de diméthylformamide anhydre et poursuit l'agitation pendant 3 heures. On ajoute ensuite, en 10 minutes, 2,6 g de chlorure de butyryle et continue l'agitation pendant encore 20 heures. La suspension obtenue est versée sur 400 cm3 d'eau distillée et extraite avec 4 fois 200 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 120 cm3 d'acétonitrile. On ajoute une solution de 2,3 g d'acide oxalique dans 60 cm3 d'acétonitrile et poursuit l'agitation pendant 1 heure à une température voisine de 20° C. Le précipité formé est séparé par filtration. On obtient ainsi 8,1 g d'oxalate de butyryloxy-3 [(phényl-4 tétrahydro-1,2,3,6 pyridyl-1)-3 propyl]-2 isoindolinone-1 fondant à 160° C.

EXEMPLE 14

A une suspension de 0,8 g d'hydrure de sodium (à 50 % en suspension dans l'huile) dans 20 cm3 de diméthylformamide anhydre, on ajoute, à une température voisine de 20° C et en 10 minutes, une solution de 6 g de [(phényl-4 tétrahydro-1,2,3,6 pyridyl-1)-3 propyl]-2 hydroxy-3 isoindolinone-1 dans 70 cm3 de diméthylformamide anhydre et poursuit l'agitation pendant 1 heure et demie. On ajoute ensuite, en 10 minutes,

1,4 g de chlorure d'acétyle et continue l'agitation pendant encore 20 heures. La suspension obtenue est versée sur 70 cm3 d'eau distillée et extraite avec 4 fois 50 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 55 cm3 de méthyléthylcétone. On ajoute une solution de 1,3 g d'acide oxalique dans 20 cm3 de méthyléthylcétone et poursuit l'agitation pendant 1 heure à une température voisine de 20° C. Le précipité formé est séparé par filtration. On obtient ainsi 1,8 g d'oxalate d'acétoxy-3 [(phényl-4 tétrahydro-1,2,3,6 pyridyl-1)-3 propyl]-2 isoindolinone-1 fondant à 166° C.

EXEMPLE 15

A une suspension de 1 g d'hydrure de sodium (à 50 % en suspension dans l'huile) dans 25 cm3 de diméthylformamide anhydre, on ajoute, à une température voisine de 20° C et en 10 minutes, une solution de 7 g de [(phényl-4 tétrahydro-1,2,3,6 pyridyl-1)-3 propyl]-2 hydroxy-3 isoindolinone-1 dans 75 cm3 de diméthylformamide anhydre et poursuit l'agitation pendant 2 heures. On ajoute ensuite, en 5 minutes, 2,3 g de chlorure d'isobutyryle et continue l'agitation pendant encore 20 heures. La suspension obtenue est versée sur 100 cm3 d'eau distillée et extraite avec 4 fois 50 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 45 cm3 de méthyléthylcétone. On ajoute une solution de 1,5 g d'acide oxalique dans 35 cm3 de méthyléthylcétone et poursuit l'agitation pendant 1 heure à une température voisine de 20° C. Le précipité formé est séparé par filtration et recristallisé dans 200 cm3 de méthanol bouillant. On obtient ainsi 3 g d'oxalate d'isobutyryloxy-3 [(phényl-4 tétrahydro-1,2,3,6 pyridyl-1)-3 propyl]-2 isoindolinone-1 fondant à 164° C.

EXEMPLE 16

A une suspension de 1 g d'hydrure de sodium (à 50 % en suspension dans l'huile) dans 25 cm3 de diméthylformamide anhydre, on ajoute, à une température voisine de 20° C et en 10 minutes, une solution de 7 g de [(phényl-4 tétrahydro-1,2,3,6 pyridyl-1)-3 propyl]-2 hydroxy-3 isoindolinone-1 dans 75 cm4 de diméthylformamide anhydre et poursuit l'agitation pendant 2 heures. On ajoute ensuite, en 5 minutes, 3,5 g de chlorure de phénylacétyle et continue l'agitation pendant encore 20 heures. La suspension obtenue est versée sur 100 cm3 d'eau distillée et extraite avec 4 fois 50 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 30 cm3 de méthyléthylcétone. On ajoute une solution de 1,1 g d'acide oxalique dans 15 cm3 de méthyléthylcétone et poursuit l'agitation pendant 1 heure à une température voisine de 20° C. Le précipité formé est séparé par filtration. On obtient ainsi 4,2 g d'oxalate de phénylacétoxy-3 [(phényl-4 tétrahydro-1,2,3,6 pyridyl-1)-3 propyl]-2 isoindolinone-1 fondant à 134° C.

EXEMPLE 17

On porte à une température voisine de 60° C une solution agitée de 11 g de [(phényl-4 tétrahydro-1,2,3,6 pyridyl-1)-3 propyl]-2 hydroxy-3 isoindolinone-1 dans 80 cm3 d'acide acétique et on ajoute 11,3 g de zinc en poudre par petites portions en 10 minutes. La suspension obtenue est ensuite portée à une température voisine de 120° C pendant 5 heures. La suspension est évaporée à sec sous pression réduite (20 mm de mercure ; 2,7 kPa) à 80° C. Le résidu est dissous dans 250 cm3 d'eau distillée et la solution est extraite avec 4 fois 50 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le précipité formé est recristallisé dans 25 cm3 d'acétonitrile bouillant. On obtient ainsi 6,1 g de [(phényl-4 tétrahydro-1,2,3,6 pyridyl-1)-3 propyl]-2 isoindolinone-1 fondant à 104° C.

EXEMPLE 18

A une solution, dans un autoclave, de 3,6 g de formyl-2 benzoate de méthyle et de 9,6 g de (phényl-4 tétrahydro-1,2,3,6 pyridyl-1)-3 amino-1 propane dans 255 cm3 de tétrahydrofuranne, on ajoute, à une température voisine de -20° C et en 5 minutes, 5 cm3 de méthylmercaptan. On porte la solution à une température voisine de 60° C pendant 4 heures. Après refroidissement à une température voisine de 20° C, la solution est versée sur 300 cm3 de soude 1 fois normale et lavée avec 3 fois 500 cm3 d'eau distillée. L'extrait organique est séché sur sulfate de magnésium anhydre, filtré et concentré à sec sous pression réduite (20 mm de mercure ; 2,7 kPa) à 40° C. Le résidu obtenu est dissous dans 20 cm3 d'acétate d'éthyle et versé sur 750 g de silice contenue dans une colonne de 8 cm de diamètre. On élue avec de l'acétate d'éthyle. Les 3 400 premiers cm3 sont éliminés et les 3 100 cm3 suivants sont évaporés à sec sous pression réduite (20 mm de mercure ; 2,7 kPa) à 40° C. Le résidu obtenue est dissous dans 70 cm3 de méthyléthylcétone. On ajoute une solution de 1,7 g d'acide oxalique dans 20 cm3 de méthyléthylcétone et poursuit l'agitation pendant 1 heure à une température voisine de 20° C. Le précipité formé est séparé par filtration. On obtient ainsi 5,9 g d'oxalate de méthylthio-3 [(phényl-4 tétrahydro-1,2,3,6 pyridyl-1)-3 propyl]-2 isoindolinone-1 fondant à 169° C.

Le (phényl-4 tétrahydro-1,2,3,6 pyridyl-1)-3 amino-1 propane peut être préparé de la manière suivante : à une solution agitée de 41 g de [(phényl-4 tétrahydro-1,2,3,6 pyridyl-1)-3 propyl]-2 phtalimide dans 355 cm3 d'éthanol, on ajoute, en 20 minutes et à une température voisine de 20° C, 17,2 cm3 d'hydrazine

monohydratée. La solution obtenue est portée, pendant 4 heures et demie, à une température voisine de 78°C. Après refroidissement à une température voisine de 0°C, on ajoute, en 30 minutes, à la suspension formée, 82 cm3 d'acide chlorhydrique concentré et poursuit l'agitation à une température voisine de 20°C pendant 16 heures. Le précipité formé est séparé par filtration et lavé avec 3 fois 50 cm3 d'eau distillée. Aux phases aqueuses et éthanoliques réunies, on ajoute 350 cm3 de soude 1 fois normale puis extrait la solution avec 3 fois 500 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40°C. Le précipité obtenu est recristallisé dans 65 cm3 d'isopropanol bouillant. On obtient ainsi 16 g de (phényl-4 tétrahydro-1,2,3,6 pyridyl-1)-3 amino-1 propane fondant à 190°C.

Le formyl-2 benzoate de méthyle peut être préparé suivant la méthode décrite par E.L. ELIEL et A.W. BURGSTAHLER, J. Am. Chem. Soc., 71, 2251 (1949).

EXEMPLE 19

A une solution de 7,8 g de (phényl-4 tétrahydro-1,2,3,6 pyridyl-1)-3 amino-1 propane dans 90 cm3 d'acétonitrile, on ajoute, à une température voisine de 20°C et en 10 minutes, 2,5 g d'acide acétyl-2 benzoïque. On poursuit l'agitation pendant 4 heures puis on ajoute, en 30 minutes, 7,6 g de chlorhydrate de (phényl-4 tétrahydro-1,2,3,6 pyridyl-1)-3 amino-1 propane. A la suspension obtenue, on ajoute ensuite, en 5 minutes, 1,2 g de cyanoborohydrure de sodium. On poursuit l'agitation pendant 16 heures. La suspension est filtrée et le précipité est lavé avec 20 cm3 d'acétonitrile. Les extraits organiques sont réunis, dilués avec 200 cm3 de chlorure de méthylène puis lavés avec 120 cm3 d'eau distillée, séchés sur sulfate de magnésium anhydre, traités au noir animal, filtrés et concentrés à sec sous pression réduite (20 mm de mercure ; 2,7 kPa) à 40°C. Le résidu obtenu est versé sur 750 g d'alumine contenue dans une colonne de 6 cm de diamètre. On élue avec 1 000 cm3 d'acétate d'éthyle et les éluats correspondants sont éliminés. On élue ensuite avec 1 000 cm3 d'un mélange d'acétate d'éthyle et de méthanol (98-2 en volumes) et les éluats correspondants sont évaporés à sec sous pression réduite (20 mm de mercure ; 2,7 kPa) à 50°C. Le résidu obtenu est dissous dans 20 cm3 de méthyléthylcétone. On ajoute une solution de 0,3 g d'acide oxalique dans 10 cm3 de méthyléthylcétone et poursuit l'agitation pendant 1 heure à une température voisine de 20°C. Le précipité formé est séparé par filtration et recristallisé dans 80 cm3 d'acétonitrile bouillant. On obtient ainsi 0,9 g d'oxalate de méthyl-3 [(phényl-4 tétrahydro-1,2,3,6 pyridyl-1)-3 propyl]-2-isoindolinone-1 fondant à 147°C.

EXEMPLE 20

A une solution agitée, sous atmosphère inerte, de 8,6 g de [(phényl-4 tétrahydro-1,2,3,6 pyridyl-1)-3 propyl]-2 phtalimide dans 80 cm3 de tétrahydrofuranne anhydre, on ajoute, en 10 minutes et à une température voisine de 20°C, 16,5 cm3 d'une solution éthérée 3 fois normale de bromure de phénylmagnésium. On poursuit l'agitation pendant 3 heures puis ajoute, en 15 minutes, 200 cm3 d'une solution aqueuse saturée de chlorure d'ammonium en maintenant la température aux environs de 20°C. La phase aqueuse est extraite avec 4 fois 100 cm3 de chlorure de méthylène. Les extraits organiques sont réunis, séchés sur sulfate de magnésium anhydre, filtrés et évaporés à sec sous pression réduite (20 mm de mercure ; 2,7 kPa) à 40°C. Le résidu est dissous dans 10 cm3 de chlorure de méthylène et versé sur 100 g de silice contenue dans une colonne de 4 cm de diamètre. On élue avec 700 cm3 d'un mélange de chlorure de méthylène et de méthanol (98,5-1,5 en volumes) et l'éluat correspondant est évaporé à sec sous pression réduite (20 mm de mercure ; 2,7 kPa) à 40°C. Le précipité obtenue est recristallisé dans 10 cm3 d'acétonitrile bouillant. On obtient ainsi 2,6 g de [(phényl-4 tétrahydro-1,2,3,6 pyridyl-1)-3 propyl]-2 phényl-3 hydroxy-3 isoindolinone-1 fondant à 123°C.

EXEMPLE 21

A 12,4 g de [(phényl-4 tétrahydro-1,2,3,6 pyridyl-1)-3 propyl]-2 phényl-3 hydroxy-3 isoindolinone-1 et 3,8 g de cyanoborohydrure de sodium refroidis à une température voisine de -25°C, on ajoute, en 45 minutes, 60 cm3 d'acide trifluoroacétique. On poursuit l'agitation pendant 2 heures à une température voisine de 20°C. La solution est diluée avec 150 cm3 de chlorure de méthylène puis lavée avec 130 cm3 d'une solution saturée de bicarbonate aqueux. La phase organique est extraite avec 4 fois 100 cm3 de chlorure de méthylène. Les extraits organiques sont réunis, lavés avec 3 fois 50 cm3 d'une solution d'acide chlorhydrique 1 fois normale, séchés sur sulfate de magnésium anhydre, traités au noir animal, filtrés et concentrés à sec sous pression réduite (20 mm de mercure ; 2,7 kPa) à 40°C. Le résidu obtenu est dissous dans 150 cm3 de tétrahydrofuranne. On ajoute 4,5 cm3 de tétraméthyléthylènediamine et porte la solution à une température voisine de 65°C pendant 1 heure. Après refroidissement à une température voisine de 20°C, la solution est diluée dans 300 cm3 de chlorure de méthylène, lavée avec 5 fois 50 cm3 d'eau distillée. L'extrait organique est séché sur sulfate de magnésium anhydre, filtré et évaporé à sec sous pression réduite (20 mm de mercure ; 2,7 kPa) à 50°C. Le résidu obtenu est versé sur 750 g d'alumine neutre contenue dans une colonne de 6 cm de diamètre. On élue avec 2 000 cm3 d'un mélange d'acétate d'éthyle et de cyclohexane (40-60 en volumes) et les éluats correspondants sont éliminés. On élue ensuite avec 3 000 cm3 d'acétate d'éthyle et les éluats correspondants sont évaporés à sec sous pression réduite (20 mm de mercure ; 2,7 kPa) à 50°C. Le résidu obtenu est dissous dans 30 cm3 de méthyléthylcétone. On ajoute une solution de 0,7 g d'acide oxalique dans 10 cm3 de méthyléthylcétone et poursuit l'agitation pendant 1 heure à une température voisine de 20°C. Le précipité formé est séparé par filtration et recristallisé dans 240 cm3 d'acétonitrile bouillant. On obtient ainsi 1,6 g d'oxalate de phényl-3 [(phényl-4 tétrahydro-1,2,3,6 pyridyl-1)-3 propyl]-2 isoindolinone-1 fondant à

198° C.

EXEMPLE 22

A 15,2 g de [(phényl-4 tétrahydro-1,2,3,6 pyridyl-1)-3 propyl]-2 hydroxy-3 benzyl-3 isoindolinone-1 et 4,6 g de cyanoborohydrure de sodium refroidis à une température voisine de -25° C, on ajoute, en 45 minutes, 75 cm3 d'acide trifluoroacétique. On poursuit l'agitation pendant 4 heures à une température voisine de 20° C. La solution est diluée avec 150 cm3 de chlorure de méthylène puis lavée avec 200 cm3 d'une solution saturée de bicarbonate aqueux. La phase aqueuse est extraite avec 4 fois 150 cm3 de chlorure de méthylène. Les extraits organiques sont réunis, lavés avec 3 fois 50 cm3 d'une solution d'acide chlorhydrique 1 fois normale, séchés sur sulfate de magnésium anhydre, traités au noir animal, filtrés et concentrés à sec sous pression réduite (20 mm de mercure ; 2,7 kPa) à 40° C. Le résidu obtenu est dissous dans 150 cm3 de tétrahydrofuranne. On ajoute 3,1 cm3 de tétraméthyléthylènediamine et porte la solution à une température voisine de 65° C pendant 1 heure. Après refroidissement à une température voisine de 20° C, la solution est diluée dans 300 cm3 de chlorure de méthylène et lavée avec 5 fois 50 cm3 d'eau distillée. L'extrait organique est séché sur sulfate de magnésium anhydre, filtré et évaporé à sec sous pression réduite (20 mm de mercure ; 2,7 kPa) à 50° C. Le résidu obtenu est dissous dans 20 cm3 d'acétonitrile. On ajoute une solution de 0,7 g d'acide oxalique dans 20 cm3 d'acétonitrile et poursuit l'agitation pendant 1 heure à une température voisine de 20° C. Le précipité formé est séparé par filtration et recristallisé dans 280 cm3 de méthanol bouillant. On obtient ainsi 4,6 g d'oxalate de benzyl-3 [(phényl-4 tétrahydro-1,2,3,6 pyridyl-1)-3 propyl]-2 isoindolinone-1 fondant à 219° C.

La [(phényl-4 tétrahydro-1,2,3,6 pyridyl-1)-3 propyl]-2 benzyl-3 hydroxy-3 isoindolinone-1 peut être préparée de la manière suivante : à une solution agitée, sous atmosphère inerte, de 17,3 g de [(phényl-4 tétrahydro-1,2,3,6 pyridyl-1)-3 propyl]-2 phtalimide dans 100 cm3 de tétrahydrofuranne anhydre, on ajoute, en 30 minutes et à une température voisine de 20° C, 75 cm3 d'une solution de bromure de benzylmagnésium préparée à partir de 3,6 g de magnésium en tournures et de 19 g de bromure de benzyle dans 75 cm3 de tétrahydrofuranne anhydre. On poursuit l'agitation pendant 3 heures, puis ajoute, en 15 minutes, 200 cm3 d'une solution aqueuse saturée de chlorure d'ammonium en maintenant la température aux environs de 20° C. La phase aqueuse est extraite avec 5 fois 100 cm3 de chlorure de méthylène. Les extraits organiques sont réunis, séchés sur sulfate de magnésium anhydre, filtrés et évaporés sous pression réduite (20 mm de mercure ; 2,7 kPa) à 40° C. Le résidu est dissous dans 50 cm3 d'acétate d'éthyle et versé sur 400 g de silice contenue dans une colonne de 8 cm de diamètre. On élue avec 800 cm3 d'acétate d'éthyle et l'éluat correspondant est évaporé à sec sous pression réduite (20 mm de mercure ; 2,7 kPa) à 40° C. On obtient ainsi 17,8 g de [(phényl-4 tétrahydro-1,2,3,6 pyridyl-1)-3 propyl]-2 benzyl-3 hydroxy-3 isoindolinone-1 dont le Rf sur plaque de silice et dans un mélange de chlorure de méthylène et de méthanol (95-5 en volumes) est de 0,13.

EXEMPLE 23

On opère comme à l'exemple 2, mais à partir d'une solution agitée de 4,6 g d'hydroxy-3 [(hydroxy-4 phényl)-4 pipérazinyl-1)-3 propyl]-2 isoindolinone-1 dans 115 cm3 de méthanol à laquelle on ajoute, à une température voisine de 20° C et en 15 minutes, 22,7 cm3 d'acide sulfurique concentré. On poursuit l'agitation pendant 5 heures à une température voisine de 65° C. Après refroidissement de la solution à une température voisine de 0° C, on ajoute, en 1 heure, 57 cm3 d'une solution d'ammoniaque aqueux à 33 %. Le précipité formé est filtré et lavé avec 50 cm3 de méthanol. On dilue le filtrat avec 200 cm3 d'eau distillée et 50 cm3 d'une solution d'ammoniaque aqueux à 33 % et extrait avec 4 fois 100 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 20 cm3 de chlorure de méthylène et la solution est versée sur 500 g de silice contenue dans une colonne de 6 cm de diamètre. On élue avec un mélange de chlorure de méthylène et de méthanol (97-3 en volumes). Les 800 premiers cm3 sont éliminés et les 3 000 cm3 suivants sont évaporés à sec sous pression réduite (20 mm de mercure ; 2,7 kPa) à 50° C. Le résidu obtenu est dissous dans 35 cm3 de méthyléthylcétone. On ajoute une solution de 0,8 g d'acide oxalique dans 20 cm3 de méthyléthylcétone et poursuit l'agitation pendant 1 heure à une température voisine de 20° C. Le précipité formé est séparé par filtration et recristallisé dans 200 cm3 d'acétonitrile bouillant. On obtient ainsi 1,4 g d'oxalate de méthoxy-3 [((hydroxy-4 phényl)-4 pipérazinyl-1)-3 propyl]-2 isoindolinone-1 fondant à 147° C.

L'hydroxy-3 [((hydroxy-4 phényl)-4 pipérazinyl-1)-3 propyl]-2 isoindolinone-1 peut être préparé comme à l'exemple 3 pour la préparation de la [(phényl-4 tétrahydro-1,2,3,6 pyridyl-1)-3 propyl]-2 hydroxy-3 isoindolinone-1, mais à partir d'une solution agitée de 12,3 g d'[((hydroxy-4 phényl)-4 pipérazinyl-1)-3 propyl]-2 phtalimide dans 350 cm3 de méthanol et 35 cm3 d'eau distillée dans laquelle on ajoute, à une température voisine de 20° C, 1,8 g de borohydrure de potassium. On poursuit l'agitation pendant 24 heures. La solution obtenue est versée sur 200 cm3 d'eau distillée et extraite avec 4 fois 100 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40° C. Le résidu obtenu est dissous dans 30 cm3 de méthyléthylcétone bouillant. Après refroidissement à une température voisine de 20° C, le précipité formé est séparé par filtration. On obtient ainsi 4,9 g d'hydroxy-3 [((hydroxy-4 phényl)-4 pipérazinyl-1)-3 propyl]-2 isoindolinone-1 fondant à 190° C.

L'[((hydroxy-4 phényl)-4 pipérazinyl-1)-3 propyl]-2 phtalimide peut être préparé comme à l'exemple 3 pour la préparation de la [(phényl-4 tétrahydro-1,2,3,6 pyridyl-1)-3 propyl]-2 phtalimide, mais à partir d'une solution

agitée de 18 g de (bromo-3 propyl)-2 phtalimide et de 17,4 g d'(hydroxy-4 phényl)-4 pipérazine dans 300 cm3 de toluène à laquelle on ajoute, en 10 minutes et à une température voisine de 20° C, 19 cm3 de triéthylamine. Le précipité obtenu est dissous dans 50 cm3 de chlorure de méthylène et versé sur 2 000 g de silice contenue dans une colonne de 8 cm de diamètre. On élue avec un mélange de chlorure de méthylène et de méthanol (98-2 en volumes). Les 3 000 premiers cm3 sont éliminés et les éluats correspondants aux 1 500 cm3 suivants sont concentrés à sec sous pression réduite (20 mm de mercure ; 2,7 kPa) à 50° C. On obtient 12,3 g d'une huile orangée dont le Rf sur plaque de silice et dans un mélange de chlorure de méthylène et de méthanol (95-5 en volumes) est de 0,3.

La présente invention concerne également les médicaments constitués par un composé de formule (I), sous forme libre ou sous forme de sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont utiles pour le traitement des affections où la sérotonine est impliquée et notamment les affections du système nerveux central, du système cardiovasculaire et les troubles gastrointestinaux. Ils sont, en particulier, utiles pour le traitement de l'anxiété, des troubles du sommeil, de la dépression, des psychoses et notamment de schizophrénie, de la migraine, de l'asthme, de l'hypertension et de l'urticaire, comme analgésiques et comme inhibiteurs de l'agrégation plaquettaire.

Les doses dépendent de l'effet recherché, de la durée du traitement et de la voie d'administration utilisée ; elles sont généralement comprises entre 10 et 300 mg par jour par voie orale pour un adulte avec des doses unitaires allant de 2 à 100 mg de substance active.

D'une façon générale, le médecin déterminera la posologie appropriée en fonction de l'âge, du poids et de tous les autres facteurs propres au sujet à traiter.

Les exemples suivants, donnés à titre non limitatif, illustrent une composition selon l'invention :

Exemple A

On prépare, selon la technique habituelle, des gélules dosées à 50 mg de produit actif ayant la composition suivante :

| | |
|---|---|
| - Méthoxy-3 [((fluoro-4 phényl)-4 tétrahydro-1,2,3,6 pyridyl-1)-3 propyl]-2 isoindolinone-1... | 50 mg |
| - Cellulose ... | 18 mg |
| - Lactose ... | 55 mg |
| - Silice colloïdale ... | 1 mg |
| - Carboxyméthylamidon sodique ... | 10 mg |
| - Talc ... | 10 mg |
| - Stéarate de magnésium ... | 1 mg |

Exemple B

On prépare, selon la technique habituelle, des comprimés dosés à 50 mg de produit actif ayant la composition suivante :

| | |
|---|---|
| - Méthoxy-3 [((fluoro-4 phényl)-4 tétrahydro-1,2,3,6 pyridyl-1)-3 propyl]-2 isoindolinone-1 ... | 50 mg |
| - Lactose ... | 104 mg |
| - Cellulose ... | 40 mg |
| - Polyvidone ... | 10 mg |
| - Carboxyméthylamidon sodique ... | 22 mg |
| - Talc ... | 10 mg |
| - Stéarate de magnésium ... | 2 mg |
| - Silice colloïdale ... | 2 mg |
| - Mélange d'hydroxyméthylcellulose, glycérine, oxyde de titane (72-3,5-24,5) ... | q.s.p. 1 comprimé pelliculé terminé à 245 mg |

Exemple C

On prépare une solution injectable contenant 10 mg de produit actif ayant la composition suivante :

| | |
|---|---|
| - Méthyl-3 [(phényl-4 tétrahydro-1,2,3,6 pyridyl-1)-3 propyl]-2 isoindolinone-1 ... | 10 mg |
| - Acide benzoïque ... | 80 mg |
| - Alcool benzylique ... | 0,06 cm3 |
| - Benzoate de sodium ... | 80 mg |
| - Ethanol à 95 % ... | 0,4 cm3 |
| - Hydroxyde de sodium ... | 24 mg |
| - Propylène glycol ... | 1,6 cm3 |
| - Eau ... | q.s.p. 4 cm3 |

**Revendications**

1. Composés de formule :

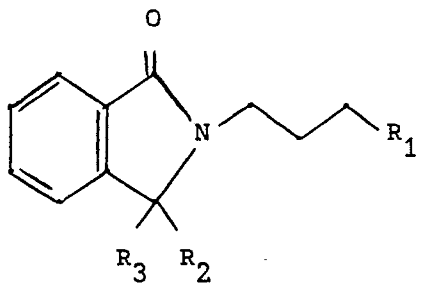

(I)

dans laquelle :- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un atome d'hydrogène ou un radical alcoxy contenant 1 ou 2 atomes de carbone, hydroxy, alkyle, alkylthio, alkylcarbonyloxy, phénylalkylcarbonyloxy, phénylalkyle ou $-NR_4R_5$ dans lequel $R_4$ représente un atome d'hydrogène ou un radical alkyle et $R_5$ représente un radical alkyle, phényle, phényle substitué par un atome d'halogène ou pyridyle et $R_3$ représente un atome d'hydrogène ou bien $R_2$ représente un radical phényle et $R_3$ représente un atome d'hydrogène ou un radical hydroxy ;
- soit $R_1$ représente un radical phényl-4 pipérazinyl-1 dont le noyau phényle est substitué en position-4 par un atome d'hydrogène ou un radical hydroxy, $R_2$ représente un radical alcoxy et $R_3$ représente un atome d'hydrogène ;
étant entendu que lorsque $R_1$ représente le radical phényl-4 tétrahydro-1,2,3,6 pyridyl-1, $R_2$ n'est pas le radical hydroxy et que, sauf mention contraire, les radicaux alkyle et alcoxy et les portions alkyle et alcoxy contiennent 1 à 4 atomes de carbone en chaîne droite ou ramifiée,
et leurs sels d'addition avec un acide minéral ou organique.

2 - Composés de formule (I) selon la revendication 1 dans laquelle :
- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un radical alcoxy, hydroxy, alkyle, alkylthio, ou alkylcarbonyloxy et $R_3$ représente un atome d'hydrogène,
- soit $R_1$ représente un radical (fluoro-4 phényl)-4 pipérazinyl-1, $R_2$ représente un radical alcoxy et $R_3$ représente un atome d'hydrogène,
étant entendu que lorsque $R_1$ représente le radical phényl-4 tétrahydro-1,2,3,6 pyridyl-1, $R_2$ n'est pas le radical hydroxy et que les radicaux alkyle et alcoxy et les portions alkyle et alcoxy contiennent 1 à 4 atomes de carbone en chaîne droite ou ramifiée, et leurs sels d'addition avec un acide minéral ou organique.

3 - Procédé de préparation des composés de formule (I) selon la revendication 1 pour lesquels $R_2$ représente un radical alcoxy et $R_1$, $R_3$ sont définis comme à la revendication 1 caractérisé en ce que l'on alkyle un dérivé de formule :

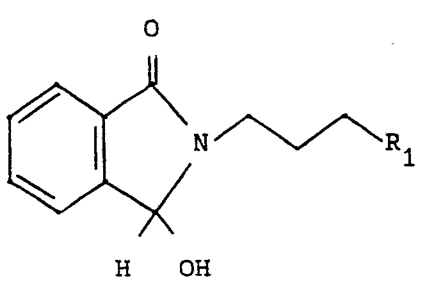

(II)

dans laquelle $R_1$ a la même signification que précédemment, puis isole le produit obtenu et la transforme éventuellement en sel d'addition avec un acide minéral ou organique.

4 - Procédé de préparation du composé de formule (I) selon la revendication 1 pour lequel $R_2$ représente un radical hydroxy et $R_1$, $R_3$ sont définis comme à la revendication 1 caractérisé en ce que l'on réduit un dérivé de formule :

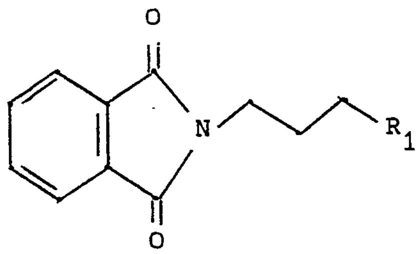

(III)

dans laquelle $R_1$ représente un radical (fluoro-4 phényl)-4 tétrahydro-1,2,3,6 pyridyl-1, isole le produit obtenu et le transforme éventuellement en sel d'addition avec un acide minéral ou organique.

5 - Procédé de préparation du composé de formule (I) selon la revendication 1 pour lequel $R_2$ représente un radical hydroxy et $R_1$, $R_3$ sont définis comme à la revendication 1 caractérisé en ce que l'on fait réagir la (bromo-3 propyl)-2 hydroxy-3 isoindolinone-1 avec une amine de formule :

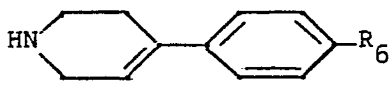

(IV)

dans laquelle $R_6$ représente un atome de fluor, isole le produit obtenu et le transforme éventuellement en sel d'addition avec un acide minéral ou organique.

6 - Procédé de préparation des composés de formule (I) selon la revendication 1 pour lesquels $R_2$ et $R_3$ représentent chacun un atome d'hydrogène et $R_1$, est défini comme à la revendication 1 caractérisé en ce que l'on réduit les composés de formule :

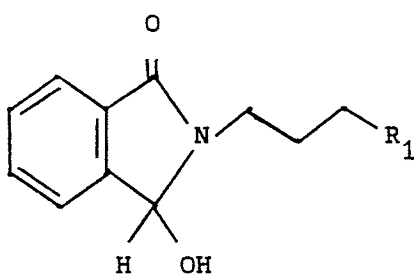

(II)

dans laquelle $R_1$ représente un radical phényl-4 tétrahydro-1,2,3,6 pyridyl-1 ou (fluoro-4 phényl)-4 tétrahydro-1,2,3,6 pyridyl-1, isole le produit obtenu et le transforme éventuellement en sel d'addition avec un acide minéral ou organique.

7- Procédé de préparation des composés de formule (I) selon la revendication 1 pour lesquels $R_2$ représente un radical alkyle et $R_1$, $R_3$ sont définis comme à la revendication 1 caractérisé en ce que l'on fait réagir un dérivé de formule :

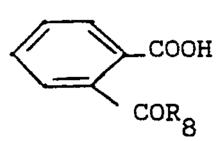

(VII)

dans laquelle $R_8$ représente un radical alkyle avec une amine de formule :

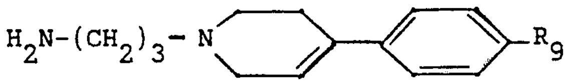

(VIII)

dans laquelle $R_9$ représente un atome d'hydrogène ou de fluor, isole le produit obtenu et le transforme éventuellement en sel d'addition avec un acide minéral ou organique.

8 - Procédé de préparation des composés de formule (I) selon la revendication 1 pour lesquels $R_2$ représente un radical alkylthio et $R_1$, $R_3$ sont définis comme à la revendication 1 caractérisé en ce que l'on fait réagir le formyl-2 benzoate de méthyle avec une amine de formule :

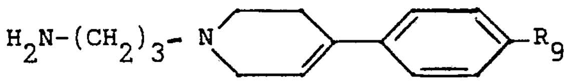

(VIII)

dans laquelle $R_9$ représente un atome d'hydrogène ou de fluor, en présence d'un alkylmercaptan, isole le produit obtenu et le transforme éventuellement en sel d'addition avec un acide minéral ou organique.

9 - Procédé de préparation des composés de formule (I) selon la revendication 1 pour lesquels $R_2$ représente un radical alcoxy, alkylcarbonyloxy ou phénylalkylcarbonyloxy et $R_1$, $R_3$ sont définis comme à la revendication 1 caractérisé en ce que l'on fait réagir un dérivé de formule :

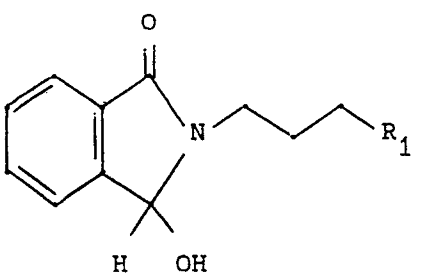

(II)

dans laquelle $R_1$ est défini comme dans la formule (I) avec un dérivé de formule :

Hal - $R_{10}$ (X)

dans laquelle Hal représente un atome d'halogène et $R_{10}$ représente un radical alkyle, alkylcarbonyle ou phénylalkylcarbonyle, isole le produit obtenu et le transforme éventuellement en sel d'addition avec un acide minéral ou organique.

10 - Procédé de préparation des composés de formule (I) selon la revendication 1 pour lesquels $R_2$ représente un radical phénylalkyle ou phényle, $R_3$ représente un atome d'hydrogène et $R_1$ est défini comme à la revendication 1 caractérisé en ce que l'on réduit un dérivé de formule :

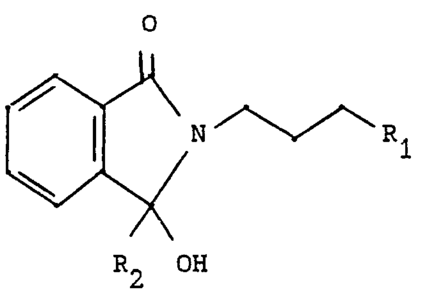

(XI)

dans laquelle $R_1$ a les mêmes significations que dans la formule (I) et $R_2$ représente un radical phényle ou phénylalkyle, isole le produit obtenu et le transforme éventuellement en sel d'addition avec un acide minéral ou organique.

11 - Procédé de préparation des composés de formule (I) selon la revendication 1 pour lesquels $R_2$ représente un radical phényle, $R_3$ représente un radical hydroxy et $R_1$ est défini comme à la revendication 1 caractérisé en ce que l'on fait réagir un halogénure de phénylmagnésium sur un dérivé de

formule :

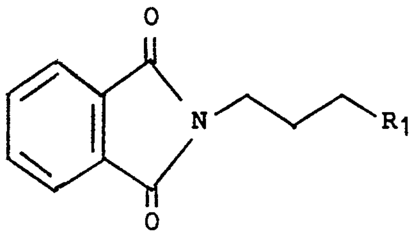

(III)

dans laquelle $R_1$ représente un radical phényl-4 tétrahydro-1,2,3,6 pyridyl-1 ou (fluoro-4 phényl)-4 tétrahydro-1,2,3,6 pyridyl-1, isole le produit obtenu et le transforme éventuellement en sel d'addition avec un acide minéral ou organique.

12 - Procédé de préparation des composés de formule (I) selon la revendication 1 pour lesquels $R_2$ représente un radical $-NR_4R_5$ et $R_1$, $R_3$, $R_4$ et $R_5$ sont définis comme dans la revendication 1 caractérisé en ce que l'on fait réagir une amine de formule :

$HNR_4R_5$ (XII)

dans laquelle $R_4$ et $R_5$ sont définis comme précédemment sur un dérivé de formule :

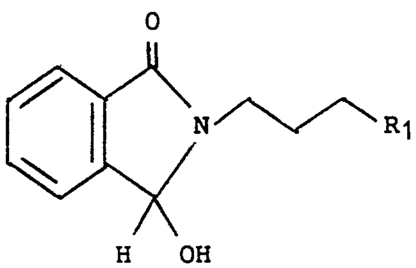

(II)

dans laquelle $R_1$ a les mêmes significations que dans la formule (I), isole le produit obtenu et le transforme éventuellement en sel d'addition avec un acide minéral ou organique.

13 - Procédé de préparation des composés de formule (I) selon la revendication 1 pour lesquels $R_2$ représente le radical $-NR_4R_5$ et $R_1$, $R_3$, $R_4$ et $R_5$ sont définis comme dans la revendication 1 caractérisé en ce que l'on fait réagir un dérivé de formule :

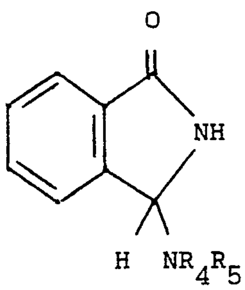

(XIII)

dans laquelle $R_4$ et $R_5$ sont définis comme précédemment avec un dérivé de formule :

$Br-(CH_2)_3-R_1$ (XIV)

dans laquelle $R_1$ est défini comme précédemment ; isole le produit obtenu et le transforme éventuellement en sel d'addition avec un acide minéral ou organique.

14 - Médicaments caractérisés en ce qu'ils contiennent, comme principe actif, au moins un composé selon la revendication 1 ou un sel d'addition avec un acide pharmaceutiquement acceptable d'un tel composé.

15 - Médicaments caractérisés en ce qu'ils contiennent, comme principe actif, au moins un composé selon la revendication 2 ou un sel d'addition avec un acide pharmaceutiquement acceptable d'un tel

composé.

**Revendications pour les Etats suivants : ES,GR**

1 - Procédé de préparation des composés de formule :

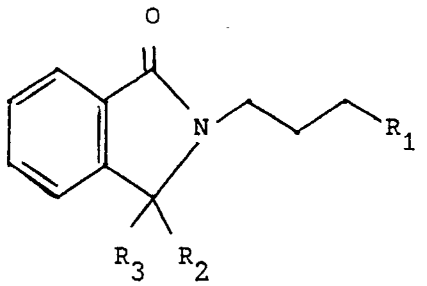

(I)

dans laquelle :
- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un atome d'hydrogène ou un radical alcoxy contenant 1 ou 2 atomes de carbone, hydroxy, alkyle, alkylthio, alkylcarbonyloxy, phénylalkylcarbonyloxy, phénylalkyle ou $-NR_4R_5$ dans lequel $R_4$ représente un atome d'hydrogène ou un radical alkyle et $R_5$ représente un radical alkyle, phényle, phényle substitué par un atome d'halogène ou pyridyle et $R_3$ représente un atome d'hydrogène ou bien $R_2$ représente un radical phényle et $R_3$ représente un atome d'hydrogène ou un radical hydroxy ;
- soit $R_1$ représente un radical phényl-4 pipérazinyl-1 dont le noyau phényle est substitué en position-4 par un atome d'hydrogène ou un radical hydroxy, $R_2$ représente un radical alcoxy et $R_3$ représente un atome d'hydrogène ;
étant entendu que lorsque $R_1$ représente le radical phényl-4 tétrahydro-1,2,3,6 pyridyl-1, $R_2$ n'est pas le radical hydroxy et que, sauf mention contraire, les radicaux alkyle et alcoxy et les portions alkyle et alcoxy contiennent 1 à 4 atomes de carbone en chaîne droite ou ramifiée,
et leurs sels d'addition avec un acide minéral ou organique caractérisé en ce que :

A - pour la préparation d'un composés de formule (I) dans laquelle $R_2$ représente un radical alcoxy et $R_1$ et $R_3$ sont définis comme précédemment, on alkyle un dérivé de formule :

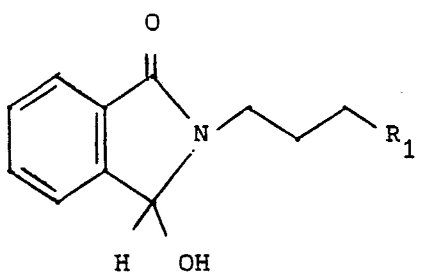

(II)

dans laquelle $R_1$ a la même signification que dans la formule (I) isole le produit obtenu et la transforme éventuellement en sel d'addition avec un acide minéral ou organique ;

B - pour la préparation d'un composé de formule (I) dans laquelle $R_2$ représente un radical hydroxy et $R_1$ et $R_3$ sont définis comme précédemment, on réduit un dérivé de formule :

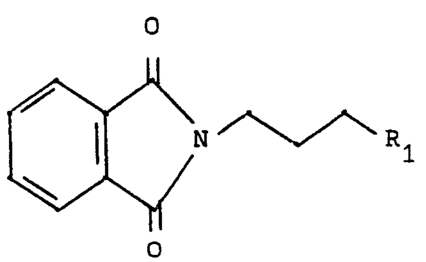

(III)

dans laquelle $R_1$ représente un radical (fluoro-4 phényl)-4 tétrahydro-1,2,3,6 pyridyl-1, isole le produit obtenu et le transforme éventuellement en sel d'addition avec un acide minéral ou organique ;

C - pour la préparation d'un composé de formule (I) dans laquelle $R_2$ représente un radical hydroxy et $R_1$ et $R_3$ sont définis comme précédemment, on fait réagir la (bromo-3 propyl)-2 hydroxy-3 isoindolinone-1 avec une amine de formule :

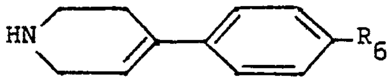

(IV)

dans laquelle $R_6$ représente un atome de fluor, isole le produit obtenu et le transforme éventuellement en sel d'addition avec un acide minéral ou organique ;

D - pour la préparation d'un composé de formule (I) dans laquelle $R_2$ et $R_3$ représentent chacun un atome d'hydrogène et $R_1$ est définis comme précédemment, on réduit un composé de formule :

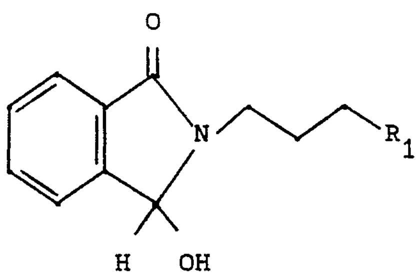

(II)

dans laquelle $R_1$ représente un radical phényl-4 tétrahydro-1,2,3,6 pyridyl-1 ou (fluoro-4 phényl)-4 tétrahydro-1,2,3,6 pyridyl-1, isole le produit obtenu et le transforme éventuellement en sel d'addition avec un acide minéral ou organique ;

E - pour la préparation d'un composé de formule (I) dans laquelle $R_2$ représente un radical alkyle et $R_1$ et $R_3$ sont défini comme précédemment, on fait réagir un dérivé de formule :

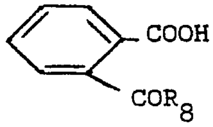

(VII)

dans laquelle $R_8$ représente un radical alkyle avec une amine de formule :

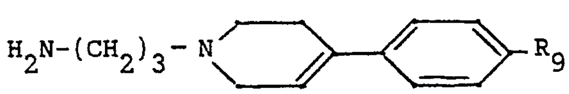

(VIII)

dans laquelle $R_9$ représente un atome d'hydrogène ou de fluor, isole le produit obtenu et le transforme éventuellement en sel d'addition avec un acide minéral ou organique ;

F - pour la préparation d'un composé de formule (I) dans laquelle $R_2$ représente un radical alkylthio et $R_1$ et $R_3$ sont définis comme précédemment, on fait réagir le formyl-2 benzoate de méthyle avec une amine de formule :

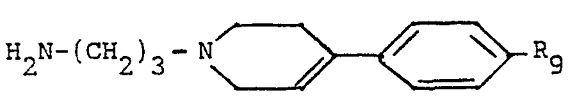

(VIII)

dans laquelle $R_9$ représente un atome d'hydrogène ou de fluor en présence d'un alkylmercaptan, isole le produit obtenu et le transforme éventuellement en sel d'addition avec un acide minéral ou

organique ;

G - pour la préparation d'un composé de formule (I) dans laquelle $R_2$ représente un radical alcoxy, alkylcarbonyloxy ou phénylalkylcarbonyloxy et $R_1$ et $R_3$ sont définis comme précédemment, on fait réagir un dérivé de formule :

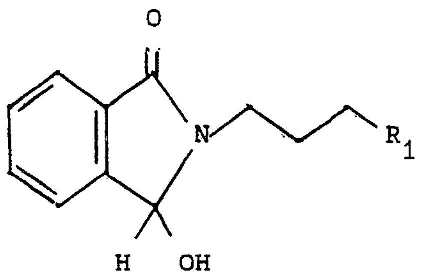

(II)

dans laquelle $R_1$ est défini comme précédemment avec un dérivé de formule :

Hal - $R_{10}$ (X)

dans laquelle Hal représente un atome d'halogène et $R_{10}$ représente un radical alkyle, alkylcarbonyle ou phénylalkylcarbonyle, isole le produit obtenu et le transforme éventuellement en sel d'addition avec un acide minéral ou organique ;

H - pour la préparation d'un composé de formule (I) dans laquelle $R_2$ représente un radical phénylalkyle ou phényle, $R_3$ représente un atome d'hydrogène et $R_1$ est défini comme précédemment, on réduit un dérivé de formule :

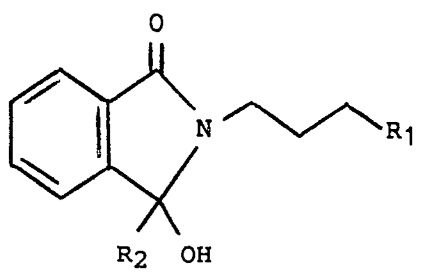

(XI)

dans laquelle $R_1$ est défini comme précédemment et $R_2$ représente un radical phényle ou phénylalkyle, isole le produit obtenu et le transforme éventuellement en sel d'addition avec un acide minéral ou organique ;

I - pour la préparation d'un composé de formule (I) dans laquelle $R_2$ représente un radical phényle, $R_3$ représente un radical hydroxy et $R_1$ est défini comme précédemment, on fait réagir un halogénure de phénylmagnésium sur un dérivé de formule :

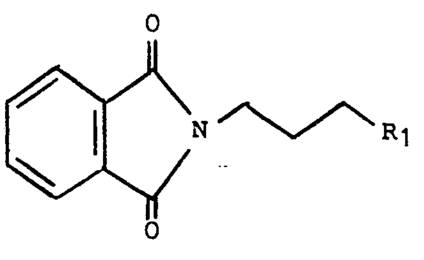

(III)

dans laquelle $R_1$ représente un radical phényl-4 tétrahydro-1,2,3,6 pyridyl-1 ou (fluoro-4 phényl)-4 tétrahydro-1,2,3,6 pyridyl-1, isole le produit obtenu et le transforme éventuellement en sel d'addition avec un acide minéral ou organique ;

J - pour la préparation d'un composé de formule (I) dans laquelle $R_2$ représente un radical $-NR_4R_5$ et $R_1$, $R_3$, $R_4$ et $R_5$ sont définis comme précédemment, on fait réagir une amine de formule :

$HNR_4R_5$ (XII)

dans laquelle $R_4$ et $R_5$ sont définis comme précédemment sur un dérivé de formule :

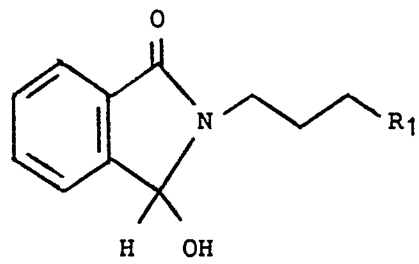

(II)

dans laquelle $R_1$ a les mêmes significations que précédemment, isole le produit obtenu et le transforme éventuellement en sel d'addition avec un acide minéral ou organique ;

K - pour la préparation d'un composé de formule (I) dans laquelle $R_2$ représente le radical $-NR_4R_5$ et $R_1$, $R_3$, $R_4$ et $R_5$ sont définis comme précédemment, on fait réagir un dérivé de formule :

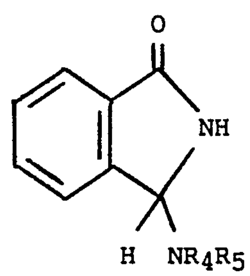

(XIII)

dans laquelle $R_4$ et $R_5$ sont définis comme précédemment avec un dérivé de formule :

$Br - (CH_2)_3 - R_1$ (XIV)

dans laquelle $R_1$ est défini comme précédemment, isole le produit obtenu et le transforme éventuellement en sel d'addition avec un acide minéral ou organique.

2 - Procédé selon la revendication 1 pour la préparation des composés de formule (I) dans laquelle :

- soit $R_1$ représente un radical phényl-4 tétrahydro-1,2,3,6 pyridyl-1 ou (fluoro-4 phényl)-4 tétrahydro-1,2,3,6 pyridyl-1, $R_2$ représente un radical alcoxy, hydroxy, alkyle, alkylthio, ou alkylcarbonyloxy et $R_3$ représente un atome d'hydrogène,
- soit $R_1$ représente un radical (fluoro-4 phényl)-4 pipérazinyl-1, $R_2$ représente un radical alcoxy et $R_3$ représente un atome d'hydrogène ;

étant entendu que lorsque $R_1$ représente le radical phényl-4 tétrahydro-1,2,3,6 pyridyl-1, $R_2$ n'est pas le radical hydroxy et que les radicaux alkyle et alcoxy et les portions alkyle et alcoxy contiennent 1 à 4 atomes de carbone en chaîne droite ou ramifiée, et leurs sels d'addition avec un acide minéral ou organique.

Office européen des brevets

# RAPPORT DE RECHERCHE EUROPEENNE

Numero de la demande

EP 89 40 0630

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-1 928 474 (MILES LABORATORIES INC.)<br>* Revendications 1,14,16; page 4 * | 1,14 | C 07 D 401/06<br>C 07 D 209/46<br>C 07 D 209/48<br>C 07 D 209/50<br>C 07 D 401/14 |
| A | GB-A-2 161 807 (BRISTOL-MYERS)<br>* Revendications 1,22 * | 1,14 | |
| | | | **DOMAINES TECHNIQUES RECHERCHES (Int. Cl.4)** |
| | | | C 07 D 401/00<br>C 07 D 209/00 |

Le présent rapport a été établi pour toutes les revendications

| Lieu de la recherche | Date d'achèvement de la recherche | Examinateur |
|---|---|---|
| LA HAYE | 30-05-1989 | CASADO Y MARTIN DE MERCA |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)